(19)

Europäisches
Patentamt
European
Patent Office
Office européen
des brevets

(11) **EP 4 492 164 A1**

(12) **EUROPEAN PATENT APPLICATION**

(43) Date of publication:
**15.01.2025 Bulletin 2025/03**

(21) Application number: **23290028.2**

(22) Date of filing: **12.07.2023**

(51) International Patent Classification (IPC):
**G05B 13/02** [(2006.01)]     **C12M 1/36** [(2006.01)]
**G01N 35/00** [(2006.01)]     **G05B 13/04** [(2006.01)]

(52) Cooperative Patent Classification (CPC):
**G05B 13/04; C12M 41/48; G05B 13/0265;**
G05B 2219/32287

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Sartorius Stedim Data Analytics AB
903 33 Umeå (SE)**

(72) Inventors:
• **Andersson, David
903 33 Umea (SE)**
• **Cloarec, Olivier
13781 Aubegne Cedex (FR)**

(74) Representative: **Müller-Boré & Partner
Patentanwälte PartG mbB
Friedenheimer Brücke 21
80639 München (DE)**

(54) **DATA DRIVEN SAMPLING METHODS FOR BAYESIAN PARAMETER ESTIMATIONS OF PHYSICAL MODELS ON PARTIAL DIFFERENTIAL EQUATIONS FOR MASS TRANSFER**

(57)     The application relates to a computer-implemented method for configuring and/or controlling a bioprocessing system that is configured to physically perform and/or simulate a bioprocess, a respective computer program product, a method for performing a bioprocess, a control device for controlling and/or configuring a bioprocessing system and a bioprocess. The method uses a probabilistic algorithm to determine a predictive posterior distribution for a target set of model parameter values and configure and/or control at least one state parameter of the bioprocessing system cand/or bioprocess based on the determined predictive posterior distribution. The probabilistic algorithm employs a combination of evaluation methods based on a common physical model, including a numerical simulation method, a search method for searching in a data storage storing a plurality precomputed solution-parameter sets of the physical model and an artificial intelligence based method using a physics informed artificial intelligence model (such as physics informed neural network model), which is trained based on the solutions of the physical model generated by the simulation method and the physical model.

Fig. 10

EP 4 492 164 A1

**Description**

The present disclosure relates to a computer-implemented method for configuring and/or controlling a bioprocessing system (such as a chromatography and/or a bioreactor system), a computer program product, a method for performing a bioprocess (such as chromatography and/or an upstream process in a bioreactor system), a control device for controlling and/or configuring a bioprocessing system, and a bioprocessing system.

A bioprocess performed on a bioprocessing system is a process for processing a biomass to obtain a target output (such as a target product) using cells and/or their components. A bioprocess typically comprises a plurality of different stages or operations, for example cell growth stage, cell harvesting stage, media preparation stage, separation stage, chromatography stage, filtering stage, stirring stage, etc.

Bioprocesses are typically complex processes that comprise a plurality of stages or processes, wherein each of the stages or processes may in turn comprise a plurality of sub-stages and so on.

For example, a bioprocess may comprise an upstream process stage (upstream process) and/or a downstream process stage (downstream process). The upstream process stage relates to the first part of a bioprocess, in which the cell culture, microbes, etc. is/are grown, for example in at least one bioreactor. An upstream process stage typically involves a plurality of steps or stages. For example, an upstream process stage may comprise culture inoculation, culture growth, fermentation, etc.

The downstream process stage typically involves processing the biomass produced by the upstream process stage to obtain a final product meeting certain quality and purity requirements. A downstream process stage typically involves a plurality of steps or stages. For example, a downstream process stage may comprise a primary recovery or clarification stage at which cells and/or debris are removed, centrifugation stage, filter stage, chromatography stage, buffer exchange stage, material hold stage, viral inactivation stage, purification stage, etc.

In a downstream process, chromatography and in particular preparative chromatography may be used to separate and purify components (e.g. chemical components) in a mixture. An aim of the chromatography and in particular of preparative chromatography is the production of a quantity of components) at the required purity, preferably with the highest output and in the most economical way.

For example, to separate a component, a mixture of components may be dissolved in a fluid medium (e.g., liquid or gas) called the mobile phase. Components in the mobile phase can then be separated by interaction with a material called the stationary phase that is usually fixed on a chromatographic bed such as a column, a capillary tube, a plate or a sheet, etc. Since different components in the mixture tend to have different affinities for the stationary phase, different components are usually retained in the chromatographic bed for different lengths of time. Thus, different components of the mixture dissolved in the mobile phase may travel through the chromatographic bed with different velocities, which allows separation of the components.

For example, in resin liquid chromatography, a solid phase (as the stationary phase) may be applied to the inner surfaces of porous resin particles, commonly spherical in shape. The particles prepared in this fashion can be packed in a cylindrical column to be able to contain the liquid mobile phase. In an initial stage of a separation process, the mobile phase may be injected or loaded into the column. As the mobile phase flows through the column, components with an affinity for the solid phase will migrate at a lower velocity. This can allow collection of highly concentrated components at the column outlet.

A chromatography process may be used for purification of a target substance, for example, a specific protein. For example, bind-elute chromatography process may be employed for capturing monoclonal antibodies using protein A in a binding matrix of a resin.

Instead of chromatography or in addition to chromatography, other separation methods such as filtering, centrifugation, etc. may be employed.

There are various challenges involved in the configuring or setup up and controlling a complex bioprocess comprising a plurality of stages or processes, each of them having one or more process parameters or settings that need to be configured, setup and/or controlled to obtain the required final product. For example, many factors and/or parameters may affect the properties of the output of a bioprocess (such as resulting yield and/or concentration and/or purity of a target component separated from other components in the mixture and/or a target component or mixture obtained at the output of a bioreactor, etc.). Further, there are complex interactions between various factors and/or parameters in the production chain. This, together with the inherent variability of the factors and/or parameters (e.g., due to manufacturing variability and/or changes over time due to usage), makes the configuration and/or control of a bioprocessing system and/or bioprocess process, in order to obtain desired results very difficult.

In order to determine a set of parameters that can lead to a desired result of a bioprocess (such as chromatography process or a bioreactor process), physical experiments of the bioprocess may be carried out. The physical experiments, however, may be time-consuming and/or expensive. Further, measurements of the properties of the output from a bioprocess (such as the compounds separated using chromatography, and in particular preparative chromatography or the bioreactor's output) is typically difficult, inaccurate and expensive. Thus, experimental data provide coarse approximations of performance at best, and after upscaling, further time and resources consuming process tuning is required. Still further, initial experiments and upscaling are costly due to lower efficiency in the production startup phase leading to loss of materials.

Alternative to or in addition to the physical experiments, in some circumstances, simulations of a bioprocess (such as chromatography process, bioreactor process, etc.) and/or bioprocessing system may be carried out. For example, to reduce cost of preparative experimentation, mass transfer modelling can be used to improve physical reasoning and predictive capability. Mass transfer within the context of the present disclosure means the movement of components (such as chemical components) in a physical system. This includes processes such as diffusive and convective mass transport, and phenomena like adsorption, desorption, precipitation, membrane filtration, etc.

Physical models may be solved with computational numerical methods and simulation software, which are known in the art. To be usable, model parameters need to be calibrated to specific process environments. Fitting experimental data in this way is typically done using computationally expensive numerical optimization. For sufficient accuracy, high-resolution calculations need to be used adding to computation time. The expense is exacerbated by the exponential growth of computation time as system size and model complexity increase.

Further, known physical models typically deterministically approximate quantities with a level of uncertainty due to several contributing factors. These factors include model assumptions and computational errors. Similarly, for model validation experimental data suffers from measurement errors and variance in material characteristics.

To account for uncertainties and to counter the deterministic nature of the models, statistical inference can be used. Instead of providing a single solution, probability distributions of model parameters may be used to generate multiple solutions from which error bounds can be determined. To further improve knowledge of a parameter distribution, experimental data can be incorporated into a statistical model. The procedure of updating knowledge with such evidence is known as Bayesian inference. However, such approach requires the simulation of the physical model to be very efficient because the algorithm used to perform such inference requires large number of samples that are typically based on Markov chains (see e.g. https://en.wikipedia.org/wiki/Markov_chain). This entails that samples are drawn sequentially, and thus individual Markov chains cannot be parallelized.

Thus, due to the complex interdependencies between various parameters of the bioprocessing system and/or bioprocess, a simulation of a bioprocess performed in a bioprocessing system typically consumes significant computational resources, such as computational time and/or power. In addition, the results of a simulation and/or experiments performed under given conditions (example in small scale) cannot be easily transferred to other conditions (example in large scale conditions).

According to an aspect, the problem relates to providing a method for configuring and/or controlling a bioprocessing system and/or a bioprocess with improved performance, and in particular reduced computational time and/or improved scalability. The problem is solved by the features disclosed by the independent claims. Further exemplary embodiments are defined by the dependent claims.

According to an aspect, there is provided a computer-implemented method for configuring and/or controlling a bioprocessing system that is configured to physically perform and/or simulate a bioprocess. Within the present disclosure the term "bioprocess" is used to refer to any process (biological and/or chemical and/or physical) which involves the processing of biomass. The term "bioprocess" may further refer to the overall bioprocess or to individual stage or stages of the overall bioprocess. Generally, it is also possible to apply the proposed method to any system performing a respective process, which can be described by a suitable physical model, in particular a physical model involving a set of (partial) differential equations.

The bioprocess may be any upstream or downstream process or a combination of an upstream and downstream bioprocesses. Non limiting examples of a bioprocesses are chromatography process, in particular a preparative chromatography process, bioreactor process, etc. Another non-limiting example of a bioprocess is a bioreactor process. Within the present disclosure, the bioprocess occurring in the one or more bioreactors or by using one or more bioreactors is referred to within the context of the present disclosure as a bioreactor process. Various types of bioreactors are known in the art, such as stirred-tank bioreactors, orbitally shaken bioreactors, wave-mixed bioreactors, etc. The form, dimensions and/or materials of the bioreactors may also greatly vary, for example from a small lab-size bioreactor to a very large bioreactor with volumes of about 2000 L and higher.

The method comprises:

determining a-posteriori parameter distribution for model parameters of a physical model of the bioprocess using a probabilistic algorithm, wherein said probabilistic algorithm uses observation data and a prior distribution of the model parameters, and wherein each model parameter corresponds to a state parameter of the bioprocessing system and/or bioprocess;
generating, using samples of the determined a-posteriori parameter distribution, a predictive posterior distribution for a target set of model parameter values (e.g. for a set of values of one or more model parameters); and
configuring and/or controlling at least one state parameter of the bioprocessing system and/or bioprocess process based on the determined predictive posterior distribution.

Further, the drawing of several samples from the determined a-posteriori parameter distribution allows the establishment of credible intervals. Accordingly, the method may further comprise generating, using samples of the determined a-posteriori parameter distribution, a predictive posterior distribution and credible interval(s) for the target set of parameter values. It is also possible to generate a plurality of predictive posterior distributions for different target sets of parameters and/or different target values.

The determining of the a-posteriori parameter distribution comprises:

(i) selecting, based on predetermined criteria, an evaluation method from among at least the following evaluation methods:

a simulation method configured to generate a solution to the physical model for a given set of values of the model parameters using a numerical method;

a search method configured to carry out a search for a nearest solution to the physical model for a given set of values of the model parameters in a data storage that stores a plurality of precomputed solution-parameter sets, each solution-parameter set comprising a precomputed solution to the physical model generated by the simulation method and the respective set of model parameter values for which the precomputed solution was generated; and

an artificial intelligence based method configured to generate a solution to the physical model for a given set of values of the model parameters using a physics informed artificial intelligence model, which is trained based on the solutions generated by the simulation method and the physical model;

(ii) drawing samples from the prior distribution of the model parameters, each sample corresponding to a complete set of sought model parameters;

(iii) for each of the samples,

- carrying out an evaluation using the currently selected evaluation method to generate an approximate solution to the physical model for the current sample,
- computing the likelihood of the generated approximate solution based on the observed data and
- combining the computed likelihood with the sample to provide a draw of the a-posteriori parameter distribution;

(iv) if the computed likelihood does not fulfill predetermined error criteria, selecting a new evaluation method from among the simulation method, the search method and the artificial intelligence based method; and

(v) iterating steps (ii) to (iv) until the a-posteriori parameter distribution reaches predetermined probabilistic algorithm criteria.

The prior distribution of the model parameters may capture the knowledge of the model parameters before observing the observation data and may include, for example value ranges or specific values for the model parameters and/or distribution(s) of model parameters. The a-posteriori parameter distribution may relate to a (e.g. normalized) conditional probability distribution onto the model parameters provided the observation data. Thus, the a-posteriori distribution for the model parameters may incorporate the evidence provided by the observation data. Given samples of the parameter a-posteriori distribution, prediction can be made by solving the model for each of the parameter sets, wherein the distribution of the predictions may be referred to as the predictive posterior distribution. The predictive posterior distribution may thus reflect the uncertainty of the physical model.

Samples may be drawn from the prior distribution of the model parameters using various methods. Exemplary methods include, but are not limited to the following methods:

drawing samples from boundaries of a domain of interest;
randomly drawing samples;
uniformly drawing samples; and/or
drawing samples through Latin Hypercube Sampling;
any other suitable sampling method.

The observation data may comprise values of at least one physical property measured in the bioprocessing system and/or at the output of the bioprocessing system. Exemplary observation data may consist of or include the yield and/or concentration of at least one component measured at a specified point or portion of a chromatography system or at a given stage of the chromatography process. The" at least one component" may be for example a target protein or other target component that is present in an output mixture of a bioreactor and/or that needs to be separated. The at least one component is not limited to a target protein or other target component, but may also include other components, such as media, buffers, waste, etc.

The "specified point" and the "specified portion" of the bioprocessing system as mentioned above may be at any point or portion within the bioprocessing system, respectively. For example, observation data may be collected at an outlet of a chromatography column or at an outlet of the chromatography system, in or at an outlet of a bioreactor or a group of bioreactors, etc.

The computer implemented method of the above aspect overcomes or alleviates one or more of the drawbacks of known methods.

In particular, employing a probabilistic algorithm enables taking error propagation in the model parameters and/or experimental data into account. Further, it is possible to easily identify correlated parameters, allowing for example design of experiments to break correlation structures. Thus, it is possible to possible to provide more accurate and realistic predictions as compared to conventional non-probabilistic methods. The proposed method has thus significant advantages over other solutions to parameter estimation for chromatography simulations, which only involve evaluating the measurement data residual in an application stage (see e.g. Heymann *et al.,* 2022) for a recent example).

Probabilistic algorithms are typically very computationally expensive and may increase the number of simulations as compared to non-probabilistic algorithms into the thousands. Due to the large iterative sampling required by probabilistic algorithms, such as MCMC algorithms, a very fast evaluation of the physical model is desirable.

Known probabilistic algorithms for complex physical models (which typically consist of or comprise a set of partial differential equations) usually employ model evaluation using suitable numerical methods. However, a numerical solution to a complex physical model (such as consisting of or comprising a set of partial differential equations) requires significant computing resources. For example, a high-fidelity model run on the most expensive consumer hardware can take seconds, meaning that a complete procedure using a probabilistic method can extend to days or weeks even for a one-dimensional model.

Thus, the model evaluation stage of a probabilistic algorithm is typically a bottleneck with respect to the performance (in particular, computation time, resources and/or resolution) of the method. Therefore, providing an alternative to on-the-fly numerical solutions can significantly decrease the time required for a probabilistic approach and improve its performance.

The proposed method relies on a combination of a several evaluation methods based on a common physical model and on switching between these methods for performing a probabilistic algorithm. The evaluation methods include a numerical simulation method, an artificial intelligence method (such as a neural network based artificial intelligence method) and a search in a data storage (such as for example an in-memory data storage). The proposed combination of evaluation methods enables a significant reduction of the evaluation time for finding a-posterior parameter distributions by using the probabilistic algorithm, thus allowing for rapid probabilistic parameter inference and prediction, in particular for a mass transfer process realized by a chromatography and/or bioreactor system and/or respective process(es). The computational gain allows the drawing of sufficient number of samples for Bayesian identification of model parameters in short time and/or with improved resolution. The a-posteriori distribution of the model parameters can be used to draw probabilistic inference on various parameters of the bioprocessing system and/or bioprocess given different experimental conditions, such as for example on the elution curves given different experimental conditions.

In addition, the proposed combination of technologies is scalable and allows a seamless transition from experiments to simulated solutions as a predictive posterior distribution from the parameter posterior distribution. This provides the user with a realistic expectation process outcome and performance. For example, simulation parameters can be known for different hardware components allowing the user to predict performance by performing experiments on one set of hardware and then switching to another pre-calibrated component. The probabilistic algorithm may have then the objective of predicting the probable outcome of performance.

More specifically, by using a search of.a data storage storing precomputed numerical solutions together with the respective model parameters for which the solutions were computed, it is possible to conserve computing resources spent on simulation, and to speed up sampling. The data storage may however have a limited capacity, thus enabling relatively course resolution of the probabilistic algorithm. By enabling a switch to a physics informed artificial intelligence method or a numerical simulation method, the resolution (and thus the accuracy) of the probabilistic algorithm may be considerably improved, while keeping the computational time reasonable. The computational time may be further reduced while maintaining high accuracy by employing an artificial intelligence based method using a physics informed artificial intelligence model (for example for sampling with an intermediate granularity) and a numerical simulation method (for example for sampling with high granularity). Thus, the very fast computation offered by the physics informed artificial intelligence model may be efficiently complemented by the high accuracy of the slower numerical simulation.

The selection of an evaluation method may be carried out according to predetermined criteria. For example, the selection of evaluation method may be constrained by the following set of guidelines:

1) an artificial intelligence model (such as a neural network models) can be trained and used as an initial approximation of the solution of the physics model. However, this approximation must generally reach a level that is good enough to perform proper probabilistic interference, e.g. proper Bayesian inference. Training of an artificial intelligence model on a range of parameters spanning an initial prior domain can be challenging because it requires a fine grid of simulated samples. This generally places a constraint on amount of data and training to reach required precision for the purpose of parameter estimation by the probabilistic method.

2) Querying pre-computed data in a data storage given a set parameter can for example outperform on-the-fly simulation method and an artificial intelligence based method depending on the size of data storage and the search algorithm used. Furthermore, the accuracy of simulated data is immutable and retains precision, in contrary to evaluations provided by the artificial intelligence based method which can be seen as a function approximator. However, depending on coarseness and boundaries of the data storage, it may not sufficiently cover required parameter space. Furthermore, probabilistic inference, such as Bayesian inference, typically requires a continuous parameter space. Sampling from the data storage is performed discretely, thus limiting the approximation of the parameter distributions. However, according to an aspect, searching from the data storage can be used in combination with the other evaluation methods to drastically reduce the total search space. This can be viewed as an extended burn-in step (burn-in samples are only used to narrow search space and later discarded). New simulations can further synergize by appending them to the data storage and thus allowing them to be queried and used by artificial intelligence model in training.

Thus, for example, in step (i), the search method, the artificial intelligence based method and the simulation method may be selected in this order. In other words, the probabilistic algorithm first selects a search in a database storing precomputed solution-parameter sets, each solution-parameter set comprising a precomputed solution to the physical model generated by the simulation method and the respective set of model parameter values for which the precomputed solution was generated. The evaluation by the search method may be continued as long as at least one (first) predetermined criterium is satisfied. When the at least one (first) predetermined criterium fails, the probabilistic algorithm may switch the evaluation method to an artificial intelligence based method. Similarly, the probabilistic algorithm may use the artificial intelligence based evaluation method as long as at least one (second) predetermined criterium is satisfied. If the at least one (second) predetermined criterium fails, the probabilistic algorithm may switch to a simulation method as the new evaluation method and may continue evaluation until all predetermined probabilistic algorithm criteria are satisfied.

For example, in step (iv) the new evaluation method may be selected based on the following (error) criteria:

if the currently selected method is the artificial intelligence based method and if, after a predetermined number of iterations, the deviation between a solution provided by the artificial intelligence based method and a solution provided by simulation method exceeds a predetermined threshold, select the search method as the new artificial intelligence based method;

if the currently selected method is the search method and precision criteria required by the probabilistic algorithm fail, select a simulation method as the new artificial intelligence based method.

Thus, by taking advantages of the strengths of individual methods of a combination of methods it is possible to carry out probabilistic inference and a method for configuring and/or control of a chromatography system and/or process based on the results of the probabilistic inference in a fast and efficient way, while maintaining high resolution.

The criteria for selecting and changing an evaluation method may for example include one or more of the following criteria: log Z, dlogz, in particular for the convergence level and rate of the likelihood. Here dlogz is the difference in logz compared to the previous iteration "I", wherein $dlogz = \log(Z_{(i-1)}/Z_i)$. For example, in case the employed MCMC algorithm is Nested Sampling, the method may focus on the evaluation of the marginal likelihood (also called evidence) and noted Z (for numerical reasons typically log(Z) is calculated), the posterior distribution of the parameters being computed during the course of the algorithm. Thus, a suitable criterial for searching and changing the evaluation method may be the incrementation of the evaluation of log(Z).

In some examples, the physics informed artificial intelligence model may be retrained using new samples generated by the simulation method. In addition or alternatively, the new samples generated by the simulation method may be optionally appended to data storage. Thus, of the search method and/or the artificial intelligence method may be improved in terms of capabilities and accuracy.

Accordingly, the method for configuring and/or controlling a bioprocessing system and/or process may further comprise:

if the currently selected method is a simulation method, updating the data storage with at least one additional solution-parameter set comprising a solution to the physical model generated by the simulation method and the respective set of model parameters for which the solution was generated; and/or

if the currently selected method is a simulation method, retraining the physics informed artificial intelligence model based on at least one solution generated by the simulation method and the physical model.

In some examples, such as when the probabilistic algorithm comprises a nested sampling probabilistic algorithm, the determining of the a-posteriori parameter distribution may comprise (e.g. after step (iii)), a step of keeping the current sample in a pool of evaluated samples or removing the current sample from a pool of evaluated samples (also referred to as "live samples") depending on the computed likelihood value. For example, if the likelihood value is higher than a threshold value, the sample may be kept.

More specifically, the method may comprise a determination whether the current sample is a part of the so-called "live samples" or, not depending on the value of its likelihood value. The number of live samples may be fixed during iterations and at each iteration the sample with the smallest likelihood may be removed from the live samples and a search of a sample with a likelihood greater than the one removed may start. Once such a sample is found, all the samples (including the new one) are ranked against their likelihood value and once more the sample with the lowest likelihood is removed from the live samples.

The determining of the a-posteriori parameter distribution may further comprise parsing observation data into a numerical representation with common unit basis of the simulation method, the search method, and the artificial intelligence based method. Thus, the processing may be facilitated and conversion errors reduced or avoided.

Bioprocessing system and/or bioprocess

The bioprocessing system may be any system configured to perform and/or simulate a bioprocess. For example, the bioprocessing system may be a chromatography system, in particular a preparative chromatography system, for performing or simulating a (preparative) chromatography process. Non-limiting examples of chromatography processes and respective systems may include, but are not limited to, a resin liquid chromatography process, a high pressure liquid chromatography process/system, a gas chromatography process/system, an ion exchange chromatography process, an affinity chromatography process/system, a membrane chromatography process/system, a continuous chromatography process/system, etc. The bioprocessing system may also be a bioreactor system for performing and/or simulating a bioreactor process, said bioreactor system comprising one or more bioreactors, such as stirred-tank bioreactors, orbitally shaken bioreactors, wave-mixed bioreactors, etc.

In some exemplary embodiments, the bioprocessing system may comprise at least one bioprocessing device configured to physically perform the bioprocess or a part of it.

For example, a bioprocessing device comprised in a chromatography system (chromatography device) may consist of or comprise one or more chromatographic beds, vessels for different kinds of media used in the chromatography process, connections between the vessels and/or the chromatographic bed(s), pumps to make the media flow through the chromatography system and/or control valves to control fluid flow and other components.

A bioprocessing device comprised in a bioreactor system may consists of or comprise one or more bioreactors, such as for example stirred-tank bioreactors, orbitally shaken bioreactors, tanks or other vessels for different media used in an upstream process, pumps to make the media flow through the bioreactor system, connections between the tanks and/or the bioreactors, control valves to control fluid flow pumps, stirring or rocking devices, and/or other components.

Accordingly, in some exemplary embodiments, the "one or more components present in the bioprocessing system" may include one or more components flowing through one or more of the connections between the vessels, chromatographic beds, bioreactors, etc. and/or flowing through the one or more vessels, chromatographic beds, bioreactors, etc. Alternatively or in addition, the "one or more components present in the bioprocessing system" may include one or more components inside one or more of the vessels, bioreactors, chromatographic beds, etc.

In the present disclosure, the term "chromatographic bed" may be understood as any of different configurations in which a stationary phase used in a chromatography process is contained. Examples of a chromatographic bed may include, but are not limited to, a column, a capillary tube, a plate, a sheet, etc.

In case the chromatography system includes a plurality of chromatographic beds, the chromatographic beds may be connected in series or in parallel or in a combination of serial and parallel connection. With a plurality of chromatographic beds connected in series, the chromatography system can perform periodic counter-current chromatography, for example.

The bioprocessing system may further comprise one or more sensors for measuring the values of one or more state parameters. The sensors may be provided at appropriate positions within the bioprocessing system. For example, in case of quantities of substances present in the bioprocessing system, ultra-violet (UV) sensors (e.g., UV-chromatogram), infrared (IR) sensors, mass spectrometry sensors, conductivity sensors (e.g., for measuring ion concentration), scatter light signal and/or Raman spectroscopy may be used for measuring the values. The sensors may further include temperature sensors, pH sensors, salinity sensors, pressure sensors, flow rate sensors or meters, etc.

Additionally or alternatively, in some circumstances, software sensors that are configured to estimate values of the state parameters from values measured by sensors may be employed. The estimated values may be derived from physical principles, data driven methods, or a combination of both.

The bioprocessing system may further comprise at least one processor and a storage medium. The processor may be configured to evaluate data and/or generate at least one instruction to other components of the bioprocessing system, such as controllers and/or generate data, such as internal state or status data. The processor may be further configured to handle input and/or or output of data and instructions to and/or from the bioprocessing system.

The processor comprised in the bioprocessing system may for example be configured to collect and/or evaluate sensor information from different kinds of sensors provided in the chromatography system and store the collected sensor information in the storage medium comprised in the bioprocessing system. The storage medium may be configured to store the collected sensor information and/or values of other parameters regarding the bioprocessing system.

Alternatively or additionally to the bioprocessing device configured to physically perform the bioprocessing process or a part thereof, the bioprocessing system may comprise a simulation system configured to simulate the bioprocessing process. The simulation system may be implemented by at least one processor and the storage medium comprised in the chromatography system, where the at least one processor is configured to perform operations and/or calculations necessary to simulate the bioprocessing process and the storage medium stores information necessary to simulate the bioprocessing process.

Further, in some exemplary embodiments, the bioprocessing system may be configured to perform a plurality of physical and/or simulated bioprocessing processes in parallel and/or in series.

In various aspects and embodiments described herein, the bioprocessing system may be configured to communicate, to another device (e.g., a control device), its internal states (e.g., represented by the one or more state parameters) at a given point in time. The communication of the internal states may be performed by the processor comprised in the bioprocessing system, for example. Further, in various aspects and embodiments described herein, the bioprocessing system may be configured to accept programmatic control to vary values of the one or more control parameters.

In the present disclosure, the one or more state parameters may be parameters representing conditions of the bioprocessing system.

In case the bioprocessing system physically performs the bioprocess, the one or more values of the one or more state parameters may be obtained by measuring the values with sensors corresponding to the state parameters. In case the bioprocessing system simulates the bioprocess, the one or more values of the one or more state parameters may be obtained by calculation performed with respect to the simulation of the bioprocess.

In various aspects and embodiments described herein, configuration and/or control of the bioprocessing system may be performed by generating one or more control signals to instruct the bioprocessing system to set the one or more state parameters to the one or more determined values. The generated control signals (e.g. by a respective control device) may then be communicated to the bioprocessing system. The bioprocessing system, in particular the at least one processor, may set and/or control the one or more control parameters to the one or more determined values, following the one or more control signals. For example, in case of controlling the position of the valve, the control signal may include a signal instructing the valve to set the valve position to the desired value. In the exemplary embodiments where the bioprocessing system comprises a bioprocessing device for physically performing the bioprocessing process or a part thereof, for example, the valve may be a control valve that is configured to vary a size of a flow passage according to the control signal. In the exemplary embodiments where the bioprocessing system comprises a simulation system, the valve may be a virtual valve and operations and/or calculations involving the virtual valve may be performed according to the position of the valve indicated by the control signal, for example.

Physical model

The physical model may be any physical model describing a bioprocess carried out on a bioprocessing system. In particular, the bioprocess may be regarded as a mass transfer process and may be modelled by a suitable physical model describing a mass transfer process in a bioprocessing system, such as for example a preparative chromatography system or a bioreactor. The physical model may be a model describing at least one physical parameter of the bioprocessing process as a function of the spatiotemporal input parameters and model parameter.

The physical model may, for example include at least one, for example more than one (i.e. a set of) partial or general differential equation(s) involving a plurality of variables (e.g. independent variables), an unknown function dependent on the variables and (partial) derivatives of the function with respect to the variables, wherein each variable corresponds to a physical property of the chromatography process.

The set of equations may relate fluid dynamics, mass transfer phenomena, mass transfer kinetics, and/or equilibrium thermodynamics. This includes, but is not limited to, models describing physical processes occurring in chromatography (see e.g. Guiochon *et al.,* 2006), stirred bioreactors (see e.g. Farsani *et al.,* 2022), wave-mixed bioreactors (see e.g. Seidel *et al.,* 2022). The set of equations is not limited to a specific set of equations but can be chosen such that the set of equations reflect for example specific hardware components, biological and/or chemical composition, physical environment and other constraints/factors regarding the bioprocess and/or the bioprocessing system on which it is performed. This includes bioprocessing devices such as pipes, pipe segments, columns, reactors, combinations of columns and reactors, vessels, bioreactors, etc. Inclusion in the physical model of additional physical phenomena that can be described using partial differential equations such as heat transfer, chemical reactions, hydrodynamical effects, is also possible.

Exemplary partial differential equation (PDE) is the convection diffusion equation (see e.g. https://en.wikipedia.org/wiki/Convection%E2%80%93diffusion_equation) which is derived from the more general continuity equation. The convection diffusion equation can be expressed as:

$$\frac{\partial y}{\partial t} + \nabla \cdot (y\boldsymbol{u}) = 0, \qquad\qquad (1)$$

where:

$y$ is a scalar field defining some conserved quantity,
$\nabla$ is the divergence operator and
$\boldsymbol{u}$ is a vector field.

The data, $y(x, t)$, may be collected from a simulated model running over timesteps $t \in \Omega_t$ over spatial coordinates $x \in \Omega_x$.

Further, a vector of problem specific parameters $\theta = (\theta_1, ..., \theta_{Np})$, for $N_p$ parameters, may be introduced. In other word, the physical model and in particular the at least one PDE may be parametrized, wherein the problem-specific parameters $\theta_1, ..., \theta_{Np}$ constitute the model parameters.

Exemplary model parameters may include, but are not limited to one or more of the following parameters: dimension parameters (e.g., column length of a chromatography column, size of a bioreactor), properties of the fluids and gases involved, properties of the equipment or consumables used in the process, such as axial dispersion coefficient, external porosity. mass transfer coefficient (e.g., lumped mass transfer coefficient, Henry coefficient, interstitial velocity, injection concentration, injection time, injection rate, gas flow rate, etc.

The proposed method is not limited to the above physical model, but can be applied in the same way for physical models that span further spatial dimensions and/or additional independent variables and/or other physical and/or chemical phenomena.

Various algorithms may be used in connection with the method for configuring and/or controlling a bioprocessing system and/or bioprocess.

Probabilistic algorithm

The probabilistic algorithm employed by the method for configuring and/or controlling a bioprocessing system may be any suitable probabilistic algorithm, such as for example a Bayesian (Bayes) inference algorithm. Bayes parameter estimation (BPE) is a technique for estimating or inferring the probability density function of random variables with unknown parameters. Exemplary Bayesian inference algorithms include but are not limited to Markov chain Monte Carlo algorithm, Nested sampling algorithm, No-U-Turn sampler algorithm, Variational autoencoder algorithm.

Specifically, parameter inference may be performed by sampling data using one or a combination of the methods (estimators):

- artificial intelligence based method (for example based on neural networks);
- data storage storing precomputed data; and
- simulation method (also referred to as "simulator" in the context of the present disclosure).

The use of the above methods will be explained further below in more detail.

Sampled data may then be used together with a general probabilistic algorithm to calculate parameter posterior distribution. The probabilistic algorithm may be based on a statistical approach based on Bayes' theorem (see e.g., https://en.wikipedia.org/wiki/Bayes%27_theorem), where the probability of an event taking place is based on prior probability distribution and conditions related to the event. Algorithms may take advantage of prior evidence in form of observation data (e.g. experimental data) to strengthen assertions about prior parameter distributions.

Algorithms for Bayesian inference include but are not limited to Markov Chain Monte Carlo or MCMC (see e.g. https://en.wikipedia.org/wiki/Markov_chain_Monte_Carlo), Nested sampling (see e.g. Skilling, 2004); No-U-Turn sampler (see e.g. Hoffman and Gelman, 2011), Variational autoencoder (see e.g. Kingma and Welling, 2014). The algorithms are based on Bayes' theorem given by the equation:

$$P(A|B) = \frac{P(A|B)P(A)}{P(B)}. \tag{2}$$

If the above equation is rewritten for a set of parameters $\theta$ and observed data $D$, the Bayes' theorem can be stated:

$$P(\boldsymbol{\theta}|D) = \frac{P(D|\boldsymbol{\theta})P(\boldsymbol{\theta})}{P(D)}. \tag{3}$$

Here the posterior probability distribution of the parameters $\theta$ is given by $P(\theta|D)$. $P(D|\theta)$ is the likelihood of observing the data given a particular parameter. $P(\theta)$ is the prior probability distribution of $\theta$, which represents the beliefs before seeing the data and $P(D)$ is the marginal likelihood, or evidence. This is the probability of observing the data averaged over all possible values of $\theta$.

Bayes' theorem allows updating the prior beliefs about the value of $\theta$ based on the observed data, by calculating the posterior distribution $P(\theta|D)$. The posterior distribution combines the prior beliefs with the likelihood of the data given the parameter values, and it provides a more informed and accurate estimate of the parameter values. The posterior distribution can then be used to simulate the predictive posterior distribution. For example, in the context of preparative chromatography parameters may be sampled and then simulated to generate a predictive posterior distribution of at least one chromatographic curve.

Simulation method

Any suitable numerical method may be used by the simulation method, in order to generate a solution to the physical model (i.e. to solve the physical model). For example, the numerical method used by the simulation method may include a known discretization method for solving partial or ordinary differential equations. For example, to transform the physical model into a numerically solvable system, the method of lines (see e.g. https://en.wikipedia.org/wiki/Method_o f_lines) may is used. The numerical method may also use a spatial discretization method such as: finite volume, finite-element, spectral or collocation method or any other suitable method to turn the physical model into a system of ordinary differential equations (see e.g. https://en.wikipedia.org/wiki/Partial_differential_equation#Numerical_solutions) in a matrix form. The system can then be solved using a temporal discretization method such as Runge-Kutta or backward-difference multistep methods (see e.g. https://en.wikipedia.org/wiki/Numerical_methods-for_ordinary_differential_equa tions).

Artificial intelligence based method

The artificial intelligence based method may employ various types of artificial intelligence models, wherein the knowledge of any physical laws that govern a given data-set (i.e. knowledge of the physical model) is used in the learning (training) process of the artificial intelligence model. Exemplary artificial intelligence models include neural networks (such as deep learning neural networks), support vector machines, k-means, kernel regression and discriminant analysis, genetic algorithms, etc.

In some examples the physics informed artificial intelligence model is a physics informed neural network model including at least one physics informed neural network.

A physics informed neural network model within the context of the present disclosure is a model, which includes one or more physics informed neural networks, such as physics informed deep learning neural networks. Physics-informed neural networks (PINNs) are a type of neural networks and may be regarded as universal function approximators that can embed the knowledge of any physical laws that govern a given dataset in the learning/training process, and can be described for example by partial differential equations (PDEs). The prior knowledge of general physical laws (i.e. the physical model) acts in the training of the neural network as a regularization agent that limits the space of admissible solutions, increasing the correctness of the function approximation. The embedding of this prior information into the neural network results in enhancing the information content of the available data, facilitating the learning algorithm to capture the right solution and to generalize well even with a low amount of training examples.

Instead of neural networks other machine learning based approaches may be employed. Similar to the neural networks, the knowledge of physical laws that govern a given dataset may be embedded in the learning process. The term "physics informed artificial intelligence model" as used within the context of the present disclosure refers not only to neural network based physics informed models but also to physics informed artificial intelligence models employing other types of machine learning methods and architectures, wherein knowledge of physical laws that govern a given data-set is embedded in the learning/training process.

To merge a physical model with a neural network or another type of a machine learning model, in order to realize a physics informed artificial intelligence model, the at least one PDE of the physical model may be expressed in the form:

$$\frac{\partial y(t,x)}{\partial t} + A[y(t,x); \zeta] = 0, \tag{5}$$

where $y$ is the latent solution and $A$ is a nonlinear operator parametrized by $\zeta$.

In the proposed method, the solutions to the physical model generated by the simulation method and optionally other data may be used together with the physical model to train a neural network or other machine-learning based algorithm which provides an approximate solution:

$$y(t,x) \approx N(t, x, \boldsymbol{\theta}_\zeta), \tag{6}$$

where $\theta_\zeta$, is a vector of parameters of a neural network with a parametrization $\zeta$. In particular, synthetic data samples $y(t, x, \theta)$ obtained by numerically solving the physical model (i.e., by a numerical simulation of the physical model) may be used to train the physics informed artificial intelligence model.

The optional other data that may be used to train the physics informed artificial intelligence model may be data obtained from other methods (e.g. other simulation methods) based on data-augmentation. Alternatively or in addition, if enough data from actual experiments is available then this data may also be used for training the physics informed artificial intelligence model.

The architecture of the physics informed artificial intelligence model can for example be created in the form of a learn solution approach (see e.g. Belbute-Peres, Chen and Sha, 2021). For the physics informed artificial intelligence model and/or learn solution operator approach, various architectures and learn approaches may be employed.

Exemplary architecture variations include, but are not limited to:

a deep neural network (DNN), which takes coordinates and the parametrization as inputs and predicts the normalized approximate solution. This neural network may have a specified number of fully or partially connected hidden layers; a deep neural network (DNN) per dependent variable; a split neural network architecture, where the first layer is comprised of two parts and the first hidden layer or layers is/are not fully connected. The first hidden layer(s) may comprise one part in which the first hidden layer(s) encode(s) the temporal and spatial coordinates, while the other part encodes the parametrization. Subsequently, the two parts may be merged in at least one merged layer, which may be followed by several fully connected hidden layers, followed by an output layer which outputs the approximate solution.

Other architectures or a combination of any of the above architectures may also be employed. Further, instead of a neural network (such as a deep neural network) other machine-learning based architectures may be employed.

Data storage

To conserve resources spent on simulation and to speed up sampling, numerical solutions $y(t, x, \theta)$ of the physical model obtained by the simulator together with the respective set of parameters for which the solutions are computed are stored in a data storage, referred to hereinafter as grid storage or simply grid. This makes pre-computed data available for rapid querying. The precomputed values may be generated at the highest level of resolution. Depending on the size requirements the data storage can be stored on the implementation hardware itself, in some external hardware, a local or network accessible database, cloud database or similar. The database may for example be a relational database, in particular spatial database, a vector database, etc.. A spatial database is a general-purpose database (usually a relational database) that is enhanced to include spatial data that represents objects defined in a geometric space, along with tools for querying and analyzing such data.

The data storage may advantageously be realized as an in-memory data storage (data structure). For example, the data storage may be realized as an in-memory database, such as in-memory relational database, in-memory spatial database, etc.

The algorithm that may be used for querying the data storage may be a nearest neighbor search. Within the context of the present disclosure, the nearest neighbor search includes any similarity search algorithm that "looks for" i.e.

searches for, entries in the data storage that are "close" to a query. The "closeness" or the "distance" between an entry in the data storage and the query may be evaluated using various metrics and criteria. Commonly used metrics are vector distance metrics and respective criteria. Other suitable metrics may be used as well.

The algorithm used for querying the data storage may be chosen such that it can quickly narrow down a search space. This includes spatial index methods such as binary space partitioning or k-d trees (see e.g. https://en.wikipedia. org/wiki/Spatial_database#Spatial_index). A "k-d tree" (short for k-dimensional tree) is a space-partitioning data structure for organizing points in a k-dimensional space. K-d trees are useful data structures for several applications, such as searches involving a multidimensional search key (e.g. range searches and nearest neighbor searches) and creating point clouds. Other options may include other "spatial data partitioning trees", such as R-tree, M-tree. etc.

It is also possible to combine different search algorithms. For example, the data store (grid store) may be realized as a set of vector databases, which can have backends that combine various search techniques (see for example https://www.pinecone.io/learn/vector-database/).

The method may further comprise a setup phase including:

generating by using the simulation method a plurality of solutions to the physical model for different values of model parameters and storing the generated solutions to the physical model and the respective set of model parameter values for which the solution was generated in the data storage; and

training an initially untrained physics informed artificial intelligence model based on the generated solutions to the physical model generated by the simulation method and optionally other data, and the physical model.

The set-up phase may further include providing a physical model and at least one prior parameter distribution for the setup phase, wherein the prior parameter distribution for the setup phase may be the same or different from the prior parameter distribution used in the parameter estimation phase.

For example, it is possible to perform a setup phase allowing a subset of problems to be solved using the same setup. This might require the prior parameter distributions in the parameter estimation phase to be subsets of the setup prior distributions. Example: If the setup phase has a uniform distribution of the parameter $\theta$ (theta) \in [0,100], it is possible to create subproblems with $\theta_{S1}$ \in [10,20] and $\theta_{S2}$ \in [40,60] in the parameter estimation phase.

In some examples synthetic samples representing solution-parameter data sets may be generated and stored in the data storage during the setup phase according to the following method:

**Step 1:** Generate synthetic data samples $y(t, x, \theta)$ using simulation of the physical model (i.e. using a simulator). The values of parameters may for example be sampled from at least one chosen parameter distribution (prior parameter distribution for the setup phase). The chosen parameter distribution(s) may be for example chosen based on prior knowledge and may include values and/or value ranges for the model parameters. The chosen parameter distribution(s) may, for example, be stored in a suitable storage device, for example in form of a table, etc.

The sampling may include, but is not limited to, one or more of the following methods:

- From boundaries of a domain of interest.
- Uniformly;
- Randomly
- Through Latin Hypercube Sampling (LHS)
- Some other probability distribution informed by domain knowledge.

**Step 2:** Store the generated data $y(t, x, \theta)$ in a data storage which allows access to the nearest solution vector as a mapping of parameter vector, $\theta \rightarrow (\boldsymbol{x}, \boldsymbol{t})$, for example using a search or partitioning algorithm, such as a k-d tree algorithm.

An exemplary setup phase of the artificial intelligence model may include the following phases:

Initiation Phase

The initiation phase may include selecting the type and/or an architecture for the physics informed artificial intelligence model, for example from among the types and/or architectures described herein.

The artificial intelligence model (such as for example a PINN model) may be initiated on computer hardware, which may be equipped with at least one general processing unit (GPU) and/or specialized processing unit. Depending on the hardware capabilities of the system the number of networks created and trained can be varied. The computer hardware may be a part of the bioprocessing system and/or of a control device for the bioprocessing system.

The artificial intelligence model architecture (e.g. the PINN model architecture) can be created in the form of a learn solution approach (see e.g. Belbute-Peres, Chen and Sha, 2021). For the artificial intelligence model (e.g. the PINN model) and/or learn solution operator approach, various architectures may be employed.

Training/Learning Phase

After initialization, the artificial intelligence model (e.g. a PINN model) undergoes a training or learning process based on data samples generated by the simulating method (and optionally further data as described above) and the physical model. Any suitable learning approach may be utilized.

For example, the learning/training of an exemplary artificial intelligence model employing physics informed neural network model (PINN model) may comprise a loop including the following stages:

a. Generate synthetic data samples $y(t, x, \theta_\zeta)$ using simulation (by e.g. numerically solving a physical model) and/or by fetching precomputed data samples $y(t,x, \theta_\zeta)$ from a data storage. Variations of how to pick chosen points may include one or more of the following:

- From boundaries of the domain of interest;
- Randomly;
- Uniformly;
- Through Latin Hypercube Sampling (LHS);
- Based on other probability distribution informed by domain knowledge.

b. Send observation data (comprising the selected synthetic data samples and optionally further data samples, e.g., from other methods and/or experimental data samples) to input nodes of the PINN model and propagate the observation data through the layers of the neural network(s) comprised in the PINN model until reaching the output layer where a prediction $\hat{y}(t,x,\zeta)$ is generated.

c. Calculate total loss $L_{total}$ as sum of partial differential equation loss (PDE loss) $L_{PDE}$ and data loss $L_{data}$ using $L_{total} = L_{data} + L_{PDE}$.

The PINN model prediction and observation data sample may be used to calculate the data loss $L_{data}$. The function for data loss can for example be calculated as:

$$L_{data} = \sum_{i=1}^{n} \lambda_i \left( \frac{1}{N_{points}} \sum_{j=1}^{N_{points}} |y_j - \hat{y}_j|^2 \right), \tag{7}$$

wherein $\lambda_i$ indicates loss weights related to each data for each dependent variable.

The same input parameters used to generate the observation data for training the PINN may be used in the partial differential component (PDE component) of the PINN to calculate the PDE loss, for example by using the following function:

$$L_{PDE} = \frac{1}{N_{points}} \sum_{i=1}^{N_{points}} |f(t_i, x_i)|^2, \tag{8}$$

where $f$ is an expression for the PDE residual, $R$, given as $f(t, x) = y_t + A[y(t,x);\zeta] \approx R$.

d. Input the calculated total loss to a PINN optimizer which produces a new set of parameters, $\boldsymbol{\theta}_\zeta^*$, replacing $\theta_\zeta$, with the purpose of minimizing the loss.

The optimization process may for example include the following steps:

- Employ an initial optimizer based on stochastic gradient descent such as the Adam optimizer (see e.g. Kingma and Ba, 2014) to update weights and biases until the loss function gets relatively close to a minimum. Such optimizers are widely used within deep learning and can efficiently find an approximate minimum;
- Switch the optimizer to a second optimizer, such as for example L-BFGS (see e.g. Liu and Nocedal, 1989) for optimizing the network parameters until termination.
- Optionally, tune the optimizer hyperparameters manually or randomly: and
- Repeat the loop until the loss has reached a defined tolerance level.

Other learning and/or optimization algorithms may also be used.

The method for configuring and/or controlling a bioprocessing system may be used for various technical purposes, including, but not limited to root-cause investigation, process scale-up, robustness studies (such as *in-silico* robustness studies), evaluating current state of the bioprocessing system (including for example evaluating the current state in a bioprocessing device comprised in the bioprocessing system), system and/or process control, etc.

For example, the configuring and/or controlling the bioprocessing system and/or bioprocess based on the determined predictive posterior distribution may comprise:

generating a prediction of a performance (i.e. predicting a performance) of the bioprocessing system and/or bioprocess for the target set of model parameter values based on the determined predictive posterior distribution; and/or

setting at least one state parameter of the bioprocessing system and/or bioprocess to the value of the corresponding model parameter included in the target set of model parameter values; and/or

controlling at least one state parameter of the bioprocessing system and/or bioprocess based on the value of the corresponding model parameter included in the target set of model parameter values.

The predicting of the performance of the bioprocessing system and/or bioprocess may comprise predicting the yield and/or concentration and/or purity of at least one component at a specified point in the bioprocessing system or at a specified stage of the bioprocess, for example at the output of the chromatography system or a bioreactor system or after completion of an end stage of an upstream or downstream process, such as for example a bioreactor process or a chromatography process. The predicting of the performance of the bioprocessing system and/or bioprocess may be carried out by a suitably programmed computing system (prediction system) that is configured to simulate the bioprocess using the method and that is implemented by at least one processor and a storage medium.

The predicting of performance may include predicting of performance after a change of a bioprocess and/or bioprocessing system, such as for example after a change of a bioreactor process and/or system, a chromatography process and/or system, etc., wherein said change may be reflected in or may cause a change of the value of at least one model parameter. For example, predicting of performance after upscaling, exchange of some process component (such as consumable component of the chromatography system), environmental change, detecting deterioration in the quality and/or amount of at least one target product produced by the bioprocess or any other technical problems, or any other change in the biosystem and/or bioprocess. In context of chromatography, the change in the chromatography system and/or chromatography process may include, but is not limited to, changing a consumable component of chromatography system (resin, monolith, membrane), changing component specific chemical and/or physical characteristics, changes in chromatography bed scale and/or type, changes in the flow rates of at least one substance flowing in the chromatography system, etc. In context of bioreactors, the change in the bioreactor system and/or bioreactor process may include, but is not limited to, upscaling the employed bioreactor(s), change of bioreactor process parameters, such as e.g. stirring rate, flow rate, etc.

The predicted performance may be displayed on a suitable display device, for example together with the predictive posterior distribution for at least one target set of model parameter values and/or the respective a-posteriori parameter distributions and/or credible intervals.

The predicted performance may be used for example for various technical purposes, such as root-cause investigation in case of sub-optimal performance of a bioprocessing system and/or bioprocess, process scale-up, robustness and/or performance evaluation (e.g. *in-silico* robustness and/or performance evaluation) of the chromatography system and/or process, control of the bioprocessing system and/or bioprocess, etc.

If, for example, it is determined that the predicted performance meets certain criteria (for example if predicted yield and/or concentration and/or purity and/or other property is within a certain range, in particular higher than a certain threshold), the value of at least one state parameter of the biosystem and/or bioprocess may be set to the value of the corresponding model parameter included in the target set of model parameter values or may be controlled based on may be set to the value of the corresponding model parameter included in the target set of model parameter values. If it is determined that the predicted performance is not within a certain range, a respective report may be automatically generated and displayed to the user, for example together with the predicted performance, and optionally the predictive posterior distribution for at least one target set of model parameter values and/or the respective a-posteriori parameter distributions and/or credible intervals.

The setting of at least one state parameter may include calibrating, adjusting or changing of the at least one state parameter. The setting may be performed during a configuration stage (e.g., as a part of the bioprocessing system and/or bioprocess physical configuration) and during the operating of the bioprocessing system and/or bioprocess, e.g., as a part of the system and/or process control or calibration. Thus, it is possible to correctly and efficiently account for changes in the bioprocessing system and/or bioprocess. •

Further, the configuring and/or controlling at least one state parameter of the bioprocessing system and/or bioprocess based on the determined predictive posterior distribution may include sending instructions to at least one control device to set or change at least one state parameter of the chromatography system and/or process.

The at least one state parameter of the bioprocessing system and/or bioprocess may include one or more of the following state parameters:

parameters of at least one bioprocessing device configured to (physically) perform the whole or a certain part or stage of the bioprocess comprised in the bioprocessing system;

physical and/or chemical characteristics and/or quantity/amount of a consumable component of a bioprocessing system and/or bioprocess; and

physical and/or chemical characteristics at at least one specified point in the bioprocessing system.

The bioprocessing device may be for example a chromatographic bed (such as a chromatographic column), a vessel for a certain type of media used in the bioprocessing system, a pump to make the media flow through the bioprocessing system, a connection between the vessels and/or the chromatographic bed(s) and/or columns and/or bioreactors, a control valve to control fluid flow, and/or other upstream or downstream device. The parameters of the at least one bioprocessing device may include dimensions/scale of the bioprocessing device, pressure, volume, flow rate, stirring rate, type of chromatography device, processing time, time in use, etc.

The physical and/or chemical characteristics and/or quantity/amount of a consumable component of a bioprocessing system (such as a chromatography system) and/or bioprocess (such as a chromatography process) may include the physical and/or chemical characteristics and/or quantity/amount of a consumable component, such as resin, monolith, membrane, etc.

The physical and/or chemical characteristics at a specified point in the bioprocessing system (e.g. at the input and/or output of a chromatography device or a bioreactor) may include, but are not limited to one or more of the following characteristics: temperature, pressure, pH of media, flow rate (e.g. maximum flow rate) of at least one media flowing in the chromatography system; conductivity, absorption (e.g. absorption of at least one effluent), salinity, purity, protein content, concentration of contaminants (e.g. concentration of coeluting contaminants), target product concentration, types of media, etc.). The physical and/or chemical characteristics at a specified point in the bioprocessing system may relate to a specific component (e.g., compound and/or media) flowing in the bioprocessing system or to a plurality of compounds and/or media. The specific types of components (e.g., compounds and/or media) flowing in the bioprocessing system generally depend on the application of the bioprocessing system and/or bioprocess. Non-limiting examples of media include feed media, wash media and/or elute media, buffer media, used in an upstream and/or a downstream process, such as e.g., a chromatography process. Non limiting examples of compounds include proteins or other target substances, effluents, etc.

The specified point in the bioprocessing system may be at the output of the bioprocessing system, at the input and/or output of a specific bioprocessing device and or a specific stage of the bioprocess, within a bioprocessing device, etc. The physical and/or chemical characteristic may be set and/or controlled at one or at a plurality of specified points.

According to another aspect, there is provided a method for (physically) performing a bioprocess on a bioprocessing system, comprising:

configuring and/or controlling the bioprocessing system according to the method of any one of the various aspects and embodiments described herein.

As described above, the bioprocessing system may be any upstream and/or downstream processing system, such as for example a bioreactor system comprising one or more bioreactors or a chromatography system comprising one or more chromatographic beds. Non-limiting examples of different kinds of chromatography processes and respective systems may include, but are not limited to, a resin liquid chromatography process, a high pressure liquid chromatography process/system, a gas chromatography process/system, an ion exchange chromatography process, an affinity chromatography process/system, a membrane chromatography process/system, a continuous chromatography process/system, etc. Non-limiting examples of different kinds of bioreactor processes and respective bioreactors or bioreactor assemblies may include, but are not limited to, bioprocesses occurring in or with the help of reusable or single-use type bioreactors or bioreactor assemblies, bioreactors or bioreactor assemblies with various types of fluid mixing systems (e.g. such as for example stirred-tank bioreactors, orbitally shaken bioreactors, wave-mixed bioreactors), bioreactors or bioreactor assemblies etc. of various scales (e.g. from lab-scale bioreactors or bioreactor assemblies to large production scale bioreactors or bioreactor assemblies), etc..

According to a further aspect, there is provided a control device for configuring and/or controlling a bioprocessing system that is configured to physically perform and/or simulate a bioprocess, the control device comprising at least one processor configured to perform the method for configuring and/or controlling according to any one of the above-stated aspects and various embodiments and examples thereof. The at least one processor may be a suitably programmed general purpose processor or a dedicated processor optimizes for the specific tasks involved. The control device may also be realized as a group of interconnected processors, for example a cloud based group of processors. The control device may further comprise storage for storing (permanently or non-permanently) data and/or instructions used by and/or generated by the control device.

In particular, the control device may comprise the following modules:

an a-posteriori parameter distribution calculation module, configured to determine an a-posteriori parameter distribution for model parameters of a physical model of the bioprocess by using a probabilistic algorithm, wherein said probabilistic algorithm uses observation data and a prior distribution of the model parameters, and wherein each model parameter corresponds to a state parameter of the bioprocessing system and/or bioprocess;

a predictive posterior distribution generation calculation module, configured to generate, using samples of the determined a-posteriori parameter distribution, a predictive posterior distribution for a target set of model parameter values; and

a configuration and/or control module configured to configure and/or control at least one state parameter of the bioprocessing system and/or bioprocess based on the determined predictive posterior distribution.

The determination of the a-posteriori parameter distribution comprises the steps described in connection with the method for configuring and/or controlling a bioprocessing system according to any one of the above-stated aspects and various embodiments and examples thereof.

In particular, the a-posteriori parameter distribution calculation module may comprise the following sub-modules:

a selection module configured to select, based on predetermined criteria, an evaluation method from among at least the following evaluation methods:

a simulation method configured to generate a solution to the physical model for a given set of values of the model parameters using a numerical method;

a search method configured to carry out a search for a nearest solution to the physical model for a given set of values of the model parameters in a data storage that stores a plurality precomputed solution-parameter sets, each precomputed solution-parameter set comprising a precomputed solution to the physical model generated by the simulation method and the respective set of model parameter values for which the precomputed solution was generated; and

an artificial intelligence based method configured to generate a solution to the physical model for a given set of values of the model parameters using a physics informed artificial intelligence model, which is trained based on the solutions generated by the simulation method (and optionally other data) and the physical model;

a sample drawing module configured to draw samples from the prior distribution of the model parameters, each sample corresponding to a complete set sought model parameters;

an evaluation module, configured to carrying out an evaluation using a currently selected evaluation method to generate an approximate solution to the physical model for a current sample,

a likelihood computation module, configured to compute the likelihood of the generated approximate solution based on the observed data and to combine the computed likelihood with the sample to provide a draw of the a-posteriori parameter distribution;

an evaluation method change module, configured to determine whether the computed likelihood fulfills predetermined error criteria, and if it is determined that the computed likelihood does not fulfill predetermined error criteria, select a new evaluation method from among the simulation method, the search method and the artificial intelligence based method; and

a termination decision module configured to determine whether predetermined probabilistic algorithm criteria are fulfilled and to send instructions the sample drawing module, the likelihood computation module and the evaluation method change module to perform the respective computations until it is determined that the a-posteriori parameter distribution reaches predetermined probabilistic algorithm criteria.

Further, the control device may comprise or may be connected via suitable data connection line(s) to one or more of:

a data storage (also referred herein as grid storage or gird) storing numerical solutions $y(t, x, \theta)$ of the physical model obtained by the simulator together with the respective set of parameters for which the solutions;

a simulation computation module configured to generate a solution to the physical model for a given set of values of the model parameters using a numerical method;

an artificial intelligence model trained based on the solutions generated by the simulation method and the physical model;

an observation data storage storing observation data;

a prior storage storing prior distribution of the model parameters.

In addition, the control device may include one or more controllers for setting and/or controlling at least one respective state parameter of the bioprocessing system and/or bioprocess.

For example, in case the one or more control parameters include the one or more flow rates of one or more kinds of media flowing into and/or out of the following devices of the bioprocessing system (such as for example at least one of the one or more chromatographic beds of a chromatography system, at least one bioreactors of a bioreactor system, at least one of the other vessels comprised in the bioprocessing system) and/or at least one of the one or more flow controllers comprised in the bioprocessing system, control signals to control respective pumps and/or valves for adjusting the flow rates of the respective kinds of media may be generated and communicated to the bioprocessing system. The controlled pumps and/or valves may be physical pumps and/or valves in case the bioprocessing system physically performs the bioprocess and may be virtual pumps and/or valves in case the bioprocessing system simulates the bioprocess. In the present disclosure, the "flow controller" comprised in a bioprocessing system may be understood as a component that is configured to control flow rates from different sources of fluid (e.g., vessels) into a bioprocessing device (e.g., bioreactor or a chromatographic bed). Each bioprocessing device or vessel (e.g., each bioreactor of a bioreactor system, each chromatographic bed of a chromatography system) may have a corresponding flow controller to control fluid flow into the bioprocessing device, such as bioreactor or chromatographic bed, for example.

Further, in case the one or more control parameters include the temperature, pH, salinity, conductivity, etc. the control device may comprise respective controllers configured to generate control signals to control respective components in the bioprocessing system for adjusting the temperature, pH, salinity, conductivity, etc.

According to a further aspect, there is provided a system comprising a bioprocessing system that is configured to physically perform and/or simulate a bioprocess; and

the control device for configuring and/or controlling the bioprocessing system according to any one of the above-stated aspects and various embodiments thereof.

The bioprocessing system may any bioprocessing system, such as a bioprocessing system described herein in connection with the method for configuring and/or controlling a bioprocessing system. The bioprocessing system may for example be a chromatography system. Examples of different kinds of chromatography systems may include, but are not limited to, a resin liquid chromatography system, a high pressure liquid chromatography system, a gas chromatography system, an ion exchange chromatography system, an affinity chromatography system, a membrane chromatography system, a continuous chromatography system. The bioprocessing system may also be for example a bioreactor system comprising one or more bioreactors, such as for example stirred-tank bioreactors, orbitally shaken bioreactors, wave-mixed bioreactors, etc. Other types of bioprocessing systems or a combination of different systems are also possible.

The subject matter described in the application can be implemented as a method or as a system, possibly in the form of one or more computer program products comprising computer readable instructions that, when loaded and run on a computer, cause the computer to perform the method. The subject matter described in the application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the application.

In addition, subject matter described in the application can also be implemented as a system including a processor, and a memory coupled to the processor. The memory may encode one or more programs to cause the processor to perform one or more of the methods described in the application. In some examples, the system may be a general purpose computer system. In other examples, the system may be a special purpose computer system including an embedded system.

## BRIEF DESCRIPTION OF THE DRAWINGS

Details of one or more implementations are set forth in the exemplary drawings and description below. Other features will be apparent from the description, the drawings, and from the claims. It should be understood, however, that even though embodiments are separately described, single features of different embodiments may be combined to further embodiments.

**Figure 1** shows an exemplary method for configuring and/or controlling a bioprocessing system.

**Figures 2A** to **2C** show exemplary physics informed neural networks with different architectures, wherein **Figure 2A** shows an exemplary deep neural network (DNN), **Figure 2B** shows an exemplary deep neural network with a split architecture and **Figure 2C** shows an exemplary deep neural network for each independent variable of the physical model.

**Figure 3** shows a schematic overview of an exemplary setup phase of a physics informed neural network model.

**Figure 4** shows an exemplary probabilistic algorithm implemented by a nested sampling algorithm.

**Figure 5** shows a plot of the MSE error for PINN, k-d tree and simulation evaluation methods for initial benchmark runs.

**Figure 6** shows normalized histograms of the errors for PINN, k-d tree and simulation evaluation methods in initial benchmark.

**Figure 7** shows an error distribution of k-d tree queries in an initial benchmark.

**Figure 8** shows an error distribution of simulator evaluations in an initial benchmark.

**Figure 9** shows an error distribution of PINN evaluations in an initial benchmark.

**Figure 10** shows an overview of exemplary stages in an application phase of an exemplary method for configuring and/or control of a chromatography system.

**Figure 11** shows synthetic experimental data and source curve simulated from true parameters for a first exemplary case (case 1).

**Figure 12** shows synthetic experimental data and source curve simulated from true parameters for a second exemplary case (case 2).

**Figure 13** shows parameter distributions generated using PINN model with true parameters marked for the first exemplary case.

**Figure 14** shows parameter distributions generated using PINN with true parameters marked for the second exemplary case.

**Figure 15** shows synthetic experiment data overlayed with credible intervals generated using posterior probability derived from PINN for the first exemplary case.

**Figure 16** shows synthetic experiment data overlayed with credible intervals generated using posterior probability derived from PINN for the second exemplary case.

**Figure 17** shows parameter distributions generated using k-d tree with true parameters marked for the first exemplary case.

**Figure 18** shows parameter distributions generated using k-d tree with true parameters marked for the second exemplary case.

**Figure 19** shows synthetic experiment data overlayed with credible intervals generated using posterior probability derived from k-d tree for the first exemplary case.

**Figure 20** shows synthetic experiment data overlayed with credible intervals generated using posterior probability derived from k-d tree for the second exemplary case.

**Figure 21** shows parameter distributions generated using simulator with true parameters marked for the first exemplary case.

**Figure 22** shows parameter distributions generated using simulator with true parameters marked for the second exemplary case.

**Figure 23** shows synthetic experiment data of normalized concentration overlayed with credible intervals generated using posterior probability derived from a simulator for the first exemplary case.

**Figure 24** shows synthetic experiment data of normalized concentration overlayed with credible intervals generated using posterior probability derived from a simulator for the second exemplary case.

**Figure 25** shows experimental data of normalized concentration as a function of time measured at an output of a chromatography system.

**Figure 26** shows experiment data of normalized concentration overlayed with credible intervals generated using posterior probability derived from k-d tree.

**Figure 27** shows parameter distributions generated using k-d tree.

**Figure 28** shows experiment data of normalized concentration overlayed with credible intervals generated using posterior probability derived from PINN.

**Figure 29** shows exemplary parameter distributions generated using PINN.

**Figure 30** shows experiment data overlayed with credible intervals generated using posterior probability derived from a simulator.

**Figure 31** shows exemplary parameter distributions generated using simulator.

**Figure 32** shows experiment data overlayed with credible intervals generated using posterior probability from the combined usage of PINN, k-d tree and simulator.

**Figure 33** shows exemplary parameter distributions generated using combined usage of PINN, k-d tree and simulator.

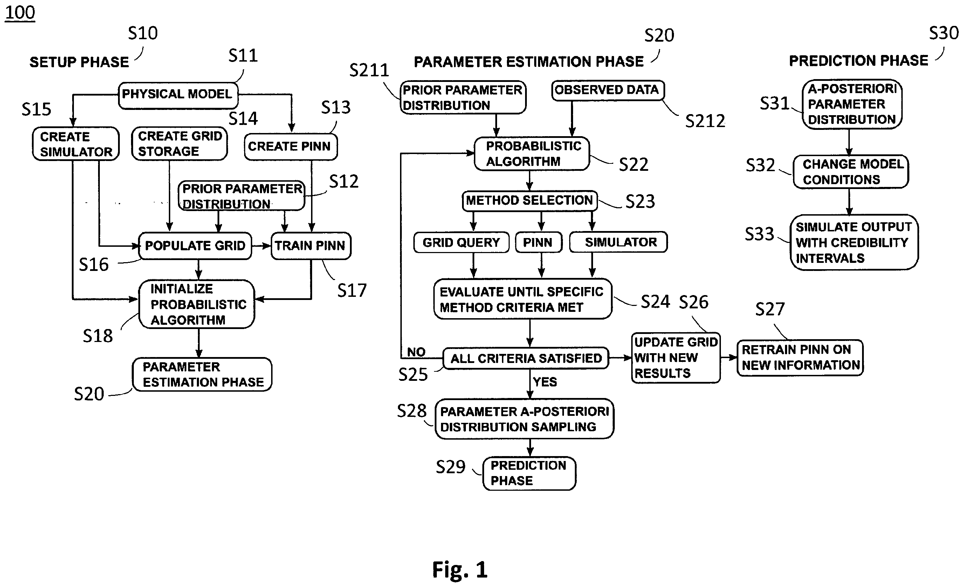

**Figure 1** shows an exemplary method 100 for configuring and/or controlling a bioprocessing system according to an aspect and its variations described herein. The method 100 comprises a three-phase process including a setup phase S10, a parameter estimation phase S20 and a prediction phase S30.

In the setup phase synthetic data is generated based on a simulation based on prior problem knowledge. The synthetic data may be generated through a numerical simulation of a physical model as described above. The synthetic data is stored in a searchable data structure, which may be a highly optimized searchable data structure. Within the context of the present disclosure the data storage storing a searchable data structure that stores data generated by a simulation (e.g. a numerical simulation) of a physical model is also referred to as the grid structure or simply grid. Furthermore, a physics informed neural network (PINN) model is trained on the synthetic data and a physical model. The physical model (including for example one or more differential equations), the PINN and the data structure (grid) are referred to within the context of the present disclosure as "evaluation methods" or "evaluators". The three evaluators are in turn used in initialization of the probabilistic algorithm and the next phase, i.e. the parameter estimation phase S20, can be started.

The setup phase may include the following steps:

In step S11 a physical model of the bioprocess performed by the bioprocessing system is provided, wherein the physical model is parametrized by a set of model parameters.

In step S12 a prior parameter distribution for the model parameters is provided, such as one of the prior parameter distributions described herein. The prior parameter distribution may be based on prior knowledge of the parameter distribution(s) of at least one model parameter.

In step S13 an initial (untrained) physics informed neural network model is provided or created, for example according to one of the methods described herein.

In step S14 an initial (for example empty) data storage (also referred herein as grid storage or grid) is provided.

In step S15, a simulation method (hereinafter also referred to as a "simulator") for numerically solving the physics method is provided.

In step S16, the data storage is populated by generating synthetic data based on a simulation of the physical model for model parameters sampled from the prior parameter distribution and storing the generated synthetic data in a searchable data storage. The synthetic data comprises or consists of a plurality of solution-parameter data set, each solution parameter data set including a solution of the physical model generated by the simulator and the respective set of model parameters for which the solution was generated.

In step S17, the initially untrained physics informed neural network model is trained based on the generated synthetic data (and optionally other data, such as data from other models and/or experimental data) and the physics model. The synthetic data may be read from the data storage and/or obtained directly from the simulator.

In step S18, a probabilistic algorithm is initialized based on the simulator, the populated data storage and the trained physics informed neural network model. The step of initializing the probabilistic algorithm may particularly include making the simulator, the populated data storage and the trained physics informed neural network model available to a probabilistic algorithm.

The method may then proceed to a parameter estimation phase S20, where either the trained physics informed neural network, the populated data storage, or the simulator are used with a probabilistic algorithm to find the a-posterior parameter distributions given some data. The probabilistic algorithm may use prior problem knowledge (e.g. in the form of a prior parameter distribution) and observation data to constrain the parameter space. To constrain the space, the probabilistic algorithm may use for example Bayesian parameter estimation together with any of the evaluation methods (evaluators) from the setup phase. Predetermined criteria may be used to select a specific evaluator, switch to a new evaluator or complete the estimation procedure. Optionally the grid can be updated with new simulated results and/or the PINN can be retrained with new information. Once all criteria are satisfied the parameter distributions are propagated to the prediction phase.
The parameter estimation phase S20 may comprise the following steps:

In step S211, prior parameter distribution is provided. The prior parameter distribution used in the parameter estimation phase S20 may be the same or different from the prior parameter distribution used on the setup phase S10. For example, the prior parameter distribution used in the parameter estimation phase S20 may be a subset of the prior parameter distribution in the setup phase. The prior parameter distribution may for example be any of the prior parameter distributions described herein.

In step S212 observation data are provided. The observation data may comprise experimental data regarding at least one physical property measured at a certain point in the bioprocessing system and/or at a certain stage of the bioprocess. Exemplary observation data may include the observation data described herein.

In step S22, a probabilistic algorithm to find the a-posterior parameter distributions given the observation data from step S212 and the prior parameter distribution from step S211 some data is started.

In step S23 an evaluation method for the probabilistic algorithm is selected. The evaluation method is selected from among the populated data storage, the trained physic informed neural network and the simulation from the setup phase. During the execution of the probabilistic algorithm the evaluation method may be changed (i.e. switched) to another evaluation method. The selection and/or switch to another evaluation method (i.e. to another evaluator) may be carried out as described above according to predetermined criteria.

In step S24, it is checked whether method specific evaluation criteria are met and if the method specific criteria are met, the evaluation continues with the currently selected evaluation method. If method specific evaluation criteria are not met, the evaluation method is switched to a new evaluation method.

In step S25, it is checked whether all criteria of the probabilistic algorithm are satisfied and if not, the execution of the probabilistic algorithm is repeated. Optionally the method may include updating (step S26) the data storage with new simulated results and/or retraining (step S27) the physics informed neural network with new information, such as new simulated results. Once all criteria are satisfied, at least one a-posteriori parameter distribution is determined (step S28) and the at least one a-posteriori parameter distribution(s) is/are propagated (step S29) to the prediction phase.

In a prediction phase S30, changes in simulation environment parameters together with samples drawn from the a-posteriori distribution are used to generate predictions and optionally credible interval(s). The prediction phase may comprise obtaining (step S31) at least one a-posteriori distribution (determined during the parameter estimation phase S20), defining model conditions (step S32) and simulating output with credible intervals (step S33) as a part of predicting the performance of the chromatography system and/or method. The defining of model conditions may include or consists of changing model conditions including changing of at least one model parameter, for example to reflect upscaling, change in environmental conditions, change in use conditions (e.g. to reflect change due to certain usage duration), change in properties of consumable materials, etc.
The method may further comprise at least one further phase (not shown), wherein based on the results of the prediction phase and in particular on the determined predictive posterior distribution at least one state parameter of the bioprocessing system and/or bioprocess is configuring and/or controlled, for example in the manner described above.

Physical model

In the above exemplary method, the physical model may be one of the physical models described herein. For example, the physical model may comprise at least one, for example more than one (i.e. a set of at least two) partial differential equations (PDE), which may involve two or more independent variables, an unknown function dependent on those variables, and one or more partial derivatives of the function with respect to the variables. An exemplary PDE is the convection diffusion equation, which is derived from the more general continuity equation. The convection diffusion equation can be expressed for example as the above equation (1).

The data (the solutions to the model) $y(x, t)$ may be collected from a simulated model running over timesteps $t \in \Omega_t$ over spatial coordinates $x \in \Omega_x$.

Further, a vector of problem specific parameters $\theta = (\theta_1, ..., \theta_{Np})$, for $N_p$ parameters, may be introduced. In other word, the physical model and in particular the at least one PDE may be parametrized, wherein the problem-specific parameters $\theta_1, ..., \theta_{Np}$ constitute the model parameters.

The proposed method is not limited to the above physical model but can be applied in the same way for physical models that span further spatial dimensions and/or additional independent variables.

Simulator

In the above example, the simulator may be any known simulator that provides a numerical solution to the physical model, i.e. to the one or more partial differential equations. The simulator may be for example any of the simulators described herein and may use for example a method of lines to transform the physical model into a numerically solvable system, which may be then solved using a known temporal discretization method such as Runge-Kutta or a backward-difference multistep method.

Data storage (grid storage)

To conserve resources spent on simulation and to speed up sampling, numerical solutions $y(t, x, \theta)$ of the physical model obtained by the simulator for a specific parameter set $\theta$ are stored in memory in a data storage, referred to hereinafter as grid storage or simply grid.

In some examples synthetic samples $y(t, x, \theta)$ may be generated using numerical simulation of the physical model by the simulator. The values of parameters may for example be sampled from chosen distributions including. The sampling may include, but is not limited to, one or more of the following methods: (i) from boundaries of a domain of interest, (ii) randomly, (iii) uniformly, (iv) through Latin Hypercube Sampling (LHS) or (v) through some other probability distribution sampling informed by domain knowledge.

The generated data $y(t, x, \theta)$ may be stored in a data storage grid which allows access to the nearest solution vector as a mapping of parameter vector, $\theta \rightarrow (x, t)$, for example using a search or partitioning algorithm. The generated data $y(t, x, \theta)$ may further be used to train a physics informed neural network model (PINN model).

This makes pre-computed data available for rapid querying. Depending on the size requirements the grid can be stored on the implementation hardware itself, in some external hardware, a local or network accessible database, cloud database or similar. The database may for example be a relational database, in particular spatial database. The grid storage may advantageously be realized in as an in-memory data-structure, for example an in-memory database (such as in-memory relational database, in-memory spatial database, etc.).

The algorithm used for querying the grid may be chosen such that it can quickly narrow down a search space. This includes spatial index methods such as binary space partitioning or k-d trees.

Physics informed neural network model

A physics informed neural network model within the context of the present disclosure is a model, which includes one or more physics informed neural networks, such as one or more of the physics informed neural networks described herein. The prior knowledge of general physical laws (i.e. the physical model) acts in the training of the neural network as a regularization agent that limits the space of admissible solutions, increasing the correctness of the function approximation. The embedding of this prior information into the neural network results in enhancing the information content of the available data, facilitating the learning algorithm to capture the right solution and to generalize well even with a low amount of training examples.

Instead of neural networks other machine learning based approaches may be employed. Similar to the neural networks, the knowledge of physical laws that govern a given dataset may be embedded in the learning process.

To merge a physical model with a neural network or another type of a machine learning model, in order to realize a PINN model, the at least one PDE of the physical model may be expressed in the form governed by the above equation (5).

In the exemplary method shown in Figure 1, the simulation is used together with the physical model to train a neural network or other machine-learning based algorithm which provides an approximate solution $y(t, x) \approx N(t, x, \theta_\zeta)$, where $\theta_\zeta$, is a vector of parameters of a neural network with a parametrization $\zeta$.

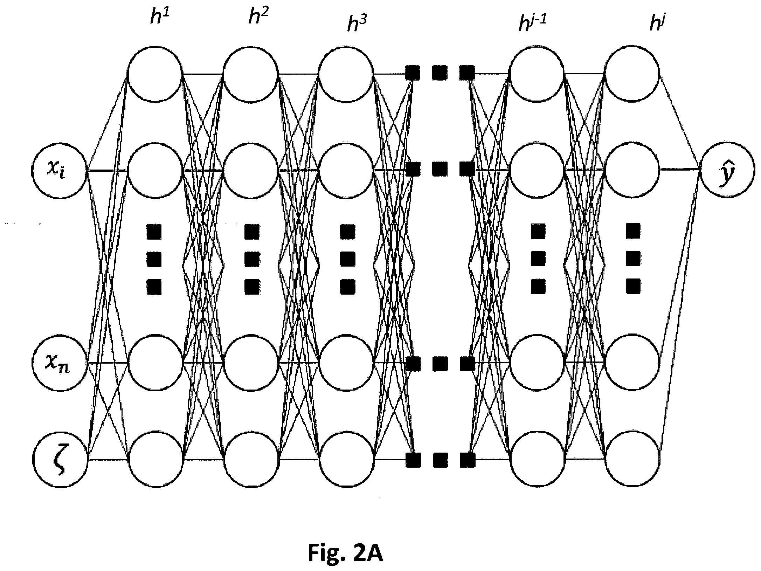

The employed neural network models may have various architectures. **Figures 2A** to **2C** show exemplary physics informed neural networks with different architectures that may be employed in the exemplary method for configuring and/or setting a bioprocessing system, such as in the exemplary method shown in Figure 1.

**Figure 2A** shows an exemplary deep neural network (DNN), which is a regular DNN with j fully connected hidden layers $h^1$ to $h^j$, wherein the DNN takes coordinates $x_i, ..., x_n$ and the parametrization $\xi$ as inputs and returns an approximate solution $\hat{y}$ as output at an output layer.

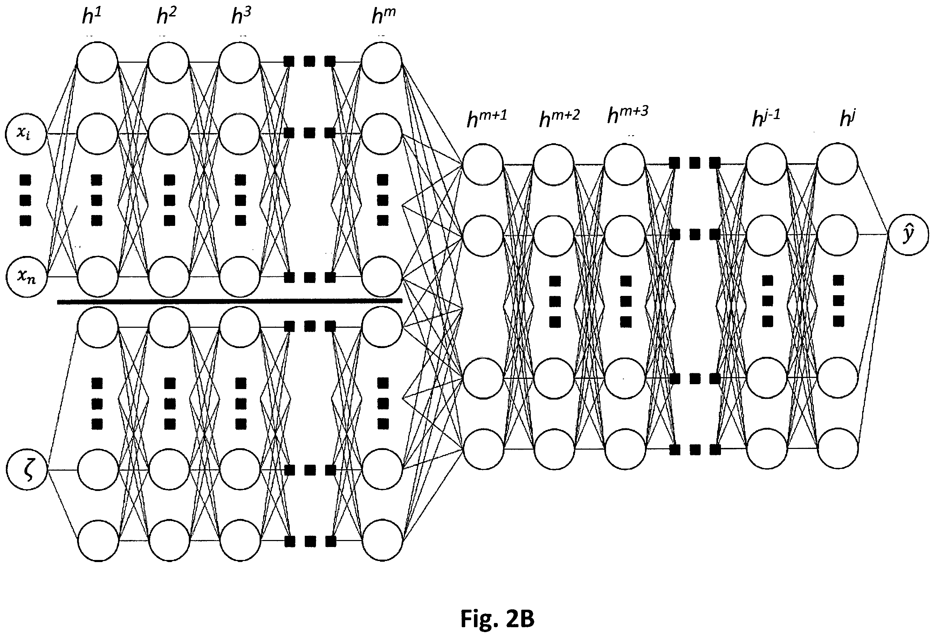

**Figure 2B** shows an exemplary deep neural network with split architecture where initial m hidden layers $h^1$ to $h^m$ encode coordinates $x_i, ..., x_n$, while the other part encodes the parametrization $\xi$. The second part merges the networks into several fully connected hidden layers $h^{m+1}$ to $h^j$, followed by an output layer which returns an approximate solution $\hat{y}$.

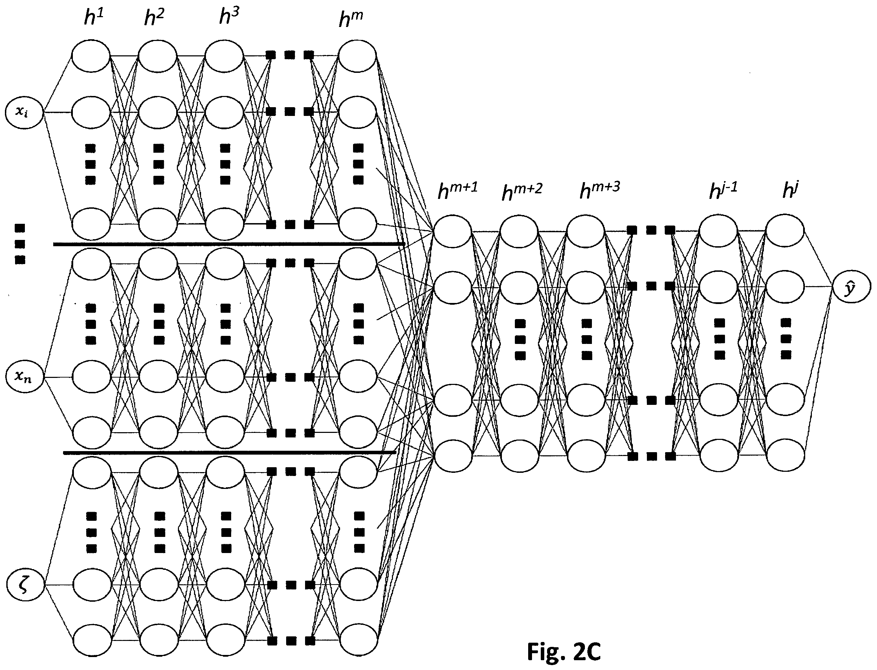

**Figure 2C** shows a neural network model comprising a plurality of deep neural networks (each having m hidden layers $h^1$ to $h^m$) for each independent variable $(x_i, ..., x_n, \xi)$, which merge into several fully connected hidden layers $h^{m+1}$ to $h^j$, followed by an output layer which returns an approximate solution $\hat{y}$.

Other architectures or a combination of any of the above architectures may also be employed. Further, instead of a neural network (such as a deep neural network) other machine-learning based architectures may be employed.

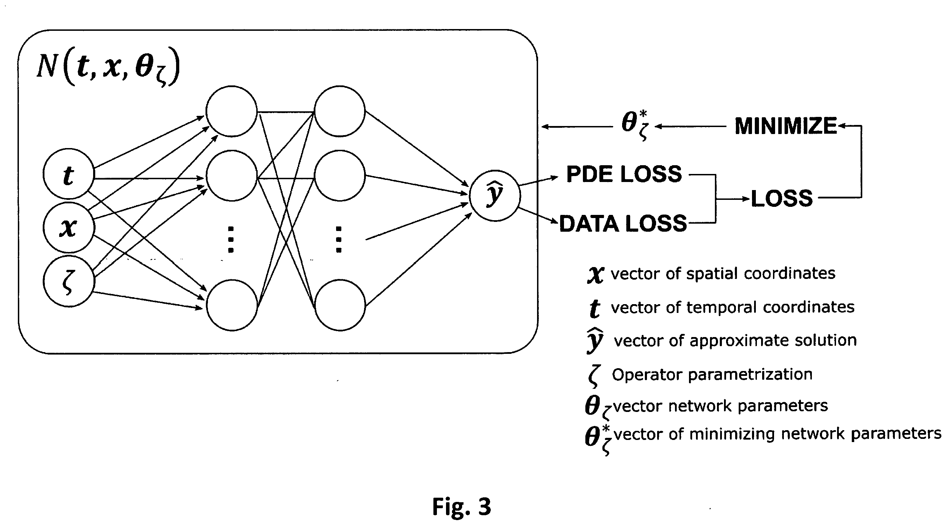

**Figure 3** shows a schematic overview of an exemplary setup phase of a physics informed neural network model.

In the setup phase a simulated data sample is generated by the simulator and the coordinates $x_i, ..., x_n$ and parameters $\xi$ used are fed to the neural network's input layer. The input layer is followed by hidden layers that make up the network architecture. The network outputs a prediction $\hat{y}$, which is used with the simulated data to calculate the data loss. The coordinates and parameters are also used to in the PDE loss function. The sum of the PDE and data loss comprise the total loss. The total loss is iteratively minimized by adjusting the parameters and feeding them back into the network.

In particular, the setup phase of the physics informed model may include an initiation phase and a training phase as described herein. In an initiation phase (e.g. step S13 in Fig. 1) physics informed neural network model (PINN model) may be initiated on computer hardware. Depending on the hardware capabilities of the system the architecture and/or number of networks created and trained can be different. The PINN model architecture can be created in the form of a learn solution approach. For the PINN model and/or learn solution operator approach, various architectures may be employed.

After initialization, the PINN model undergoes a learning/training process based on simulation results of the simulator and the physical model (e.g., step S17 in Fig. 1). For example, the physics informed neural network may go through a loop that may include the following steps:

- Generate synthetic data samples $y(t, x, \theta_\zeta)$ using simulation (by e.g. numerically solving a physical model) or fetch synthetic data samples $y(t, x, \theta_\zeta)$ from grid. Variations of how to pick chosen points may include one or more of the following: from boundaries of the domain of interest, randomly, uniformly, through Latin Hypercube Sampling (LHS), based on other probability distribution informed by domain knowledge.

- Send generated synthetic data samples to PINN input nodes and propagate the input data through the layers of the neural network until reaching the output layer where a prediction $\hat{y}(t, x, \zeta)$ is generated. Optionally, the data samples provided at the input of the PINN model may be obtained from other methods based on data-augmentation. In addition or alternatively, if enough data samples from actual experiments is available then these data samples can be also sent and used for training of the PINN could be used.

- Calculate total loss as sum of PDE and data loss using $L_{total} = L_{data} + L_{PDE}$. The PINN model prediction $\hat{y}(t, x, \zeta)$ and input synthetic data sample may be used to calculate the data loss. The function for data loss can for example be calculated as

$$L_{data} = \sum_{i=1}^{n} \lambda_i \left( \frac{1}{N_{points}} \sum_{j=1}^{N_{points}} |y_j - \hat{y}_j|^2 \right)$$

, wherein $\lambda_i$ indicates loss weights related to each data for each dependent variable. The same input parameters used to generate the observation data may be used in the PDE component of the PINN to calculate the PDE loss

$$L_{PDE} = \frac{1}{N_{points}} \sum_{i=1}^{N_{points}} |f(t_i, x_i)|^2$$

, where $f$ is an expression for the PDE residual, R, given as $f(t,x) = y_t + A[y(t,x); \zeta] \approx R$.

- Input the calculated total loss to a PINN optimizer which produces a new set of parameters, $\boldsymbol{\theta}_\zeta^*$, replacing $\theta_\zeta$, with the purpose of minimizing the loss. The optimization process may for example include the following steps:

  ◦ Employ an initial optimizer based on stochastic gradient descent such as the Adam optimizer to update weights and biases until the loss function gets relatively close to a minimum. Such optimizers are widely used within deep learning and can efficiently find an approximate minimum;

  ◦ Switch the optimizer to a second optimizer, such as for example L-BFGS for optimizing the network parameters until termination.

　○ Optionally, tune the optimizer hyperparameters manually or randomly

- Repeat the loop until the loss has reached a defined tolerance level.

Parameter estimation phase

In the parameter estimation phase, a probabilistic algorithm may be used to determine a-posteriori parameter distribution(s) and optionally confidential interval(s). In particular, Bayesian parameter estimation may be employed, wherein parameter inference may be performed by sampling data using one or a combination of the evaluation methods (evaluators):

　1) Search in a data storage storing precomputed data
　2) Trained physics informed neural network model
　3) Simulator

Sampled data may then be used together with a general probabilistic algorithm to calculate parameter posterior distribution. The probabilistic algorithm may be based on a statistical approach based on Bayes' theorem, where the probability of an event taking place is based on prior probability distribution and conditions related to the event. Algorithms may take advantage of prior evidence (in the form of observation data, such as experimental data) to strengthen assertions about prior parameter distributions. For example, in the context of preparative liquid chromatography, experimental data can consist of or comprise (normalized) component concentrations measured in or at the outlet of a chromatography column. The concentration may be determined by, but not limited to, measuring protein absorbance using 280 nm UV spectrophotometry. In the context of upstream bioprocesses (such as bioreactor processes), experimental data can consist of or comprise cell density, cell turbidity, cell viability, culture volume, total protein concentration, metabolite and/or medium concentration or other quality or target parameters measured in or at the outlet of a bioreactor or bioreactor stage. The measurements may be performed by any suitable measurement device, such as for example using spectroscopic devices.

Algorithms for Bayesian inference include but are not limited to: Markov chain Monte Carlo, Nested sampling, No-U-Turn sample, Variational autoencoder. The algorithms are based on Bayes' theorem as explained above.

An exemplary parameter estimation phase may for example include the following stages:

　1. Parsing supplied measurement data into a numerical representation, $\tilde{y}(x, t)$, with common unit basis of the simulation, grid, and PINN. This stage is optional.

　2. Selecting a simulation method for data sampling from among the three estimators provided by the setup phase, i.e. from among a populated grid storage, a trained PINN model, and simulator. In some examples the selection may be in this order, wherein the currently selected simulation method can be changed throughout the parameter estimation phase according to predetermined or predeterminable criteria of the probabilistic algorithm. The evaluation method may, for example, be chosen in the following order:

　　a. If current method is grid and no point with lower likelihood can be found, switch method to PINN;
　　b. If current method is PINN every N, steps check residual between PINN and simulator loglikelihood to a certain tolerance $e_{method}$, switch to simulator;
　　c. Numerical simulation carried out by a simulator finalizes the parameter estimation. Grid is optionally simultaneously updated with the new solutions.

　3. The currently selected evaluation method is used by the probabilistic algorithm and can include the following steps:

　　a. Algorithm draws samples from parameter prior distribution into $\theta$.
　　b. Evaluation using currently selected method given the parameters sampled in stage a. generate approximate solution $\hat{y}(x, t, \theta)$.

　　　i. Optionally, if simulator is used to generate samples append the new samples to grid.

　　c. The likelihood, a function of $f(\hat{y}, \tilde{y})$, is computed to provide a draw of the posterior distribution. The likelihood is calculated based on the observed data.
　　d. In some cases (such as for example when the probabilistic algorithm is a nested sampling probabilistic algorithm), samples may be discarded (removed) or kept in a pool of samples depending on relative likelihood value.

    e. Iterate until posterior distribution reaches probabilistic algorithm criteria.

    4. Optionally retrain PINN model on newly generated samples generated by the simulator and optionally appended to grid to update capabilities and accuracy. The accuracy of the PINN model may be determined by comparing samples drawn from the prior distribution. The exact number of samples and the exact parameter values used for the initial comparison may be defined by an experimental design.

    5. Make the determined parameter a-posteriori distribution available to a prediction phase.

As described above, a limiting stage a probabilistic method is the generation of an approximate solution may certain evaluation method (stage 3b). Typically, finding a numerical solution to a PDE or a set of PDEs requires significant computing resources in relation to common mathematical functions. However, prior art probabilistic algorithms, such as Markov-Chain Monte Carlo (MCMC) Algorithms typically require large iterative sampling and thus a very fast evaluation of the physical model.

The method for parameter estimation as described herein employes a probabilistic algorithm, wherein a combination of evaluation methods (including a simulator, PINN, and data storage storing precomputed solutions) is advantageously used to reduce or overcome the shortcomings of known probabilistic methods and/or other (e.g. deterministic methods) for parameter estimation.

Prediction phase

As described above, the prediction phase has the purpose of generating probable outcomes of changing some physical property of the simulation environment. This may include all parameters whose distributions have not been approximated in previous stages. This allows a property to be changed in the simulation together with samples drawn from the generated a posterior distribution and to generate a prediction for the model outcome. Drawing several samples may allow the generation of the distribution of the model output and thus allow the establishment of the credible intervals.

Thus, in the prediction phase, given the posterior distribution of the model parameters obtained during the parameter estimation phase, predictive posterior distribution may be generated using different experimental conditions. User may be presented with predictive posterior and credible intervals for approximate solution.

With the estimation of parameters completed, the area of application of the estimated parameters (including the a-posteriori parameter distribution(s) and optionally credible interval(s) may extend to anything that can be reasonably expected to be approximated by the underlying physical model. Applications include, but are not limited to, predicting the performance of a bioprocessing system performing a bioprocess described by the physical model derived from variations in parameters of the bioprocess and/or or bioprocessing system. This can include prediction of performance after one or more of the following: upscaling, exchange of some process component, environmental change, changes due to use over a certain time, exchanging consumable component of a chromatography system (resin, monolith, membrane, etc.) and/or of a bioreactor, variations in component specific chemical characteristics, variations in column or bioreactor scale, variations in system flow rates, etc.

The predicted performance may then be used, as described herein, to set and/or control at least one parameter of the bioprocessing system and/or bioprocess.

Further, the data storage and/or the PINN model trained by application (e.g. during the setup phase and optionally retrained during the parameter estimation phase) can be used standalone for any area where the corresponding simulator is used. This can include one or more of applications such as optimizing for production or yield by solving constraint system, evaluating current state of a chromatography system (including for example evaluating the current column state), of a bioreactor system or of any other bioprocessing system, configuring hardware elements of a bioprocessing system and/or parameters of a bioprocess executed on a bioprocessing system, controlling hardware elements of a bioprocessing system and/or parameters of a bioprocess executed on a bioprocessing system.

In the following, exemplary applications of the method and the respective system for configuring and/or controlling a bioprocessing system will be explained in further detail.

**Example**

In this example, the bioprocessing system is a chromatography system comprising at least one column.

Physical model

To model the column, a spatial variable along the axis, $z \in \Omega_z = [0, L]$, where $L$ is the column length. To advance the model in time, the time variable is t is let be $t \in [0, t_f]$, for some simulation time $t_f$.

The employed physical model describes convective mass transport and axial dispersion in the column volume with the equation:

$$\frac{\partial c}{\partial t} = -u\frac{\partial c}{\partial z} + D_{ax}\frac{\partial^2 c}{\partial z^2} - \frac{(1-\epsilon_c)}{\epsilon_c}\frac{\partial q}{\partial t}, \tag{9}$$

where

$c(t, z)$ is the mobile phase concentration,
$u$ the interstitial velocity,
$\varepsilon_c$, column porosity, and
$D_L$ axial dispersion coefficient.

To represent binding interactions between the solute component and stationary phase the Langmuir isotherm is used:

$$\frac{\partial q}{\partial t} = k_m(a \cdot c - q). \tag{10}$$

In the physical model $k_m$ denotes the lumped mass transfer coefficient and $a$, the Henry coefficient.
The inlet boundary condition is given by:

$$uc_{inj}(t) = uc(t, 0) - D_{L,i}\frac{\partial c}{\partial z}(t, 0), \tag{11}$$

where $c_{inj,i}$, defines the concentration of injected components.
The outlet boundary is defined by:

$$\frac{\partial c_i}{\partial z}(t, L) = 0. \tag{12}$$

Simulator (Simulation method)

To solve the system a numerical simulation was created by combining the equations (9) to (12) into a single semi-discrete system. The spatially dependent derivatives are discretized into $N_e$ elements using orthogonal collocation (see e.g. Young, 2019). Each variable is approximated by interpolating polynomials of order $N_p$ in each element with $C^1$-continuous boundaries. To solve the semi discrete system fixed point iteration using the backward Euler method was applied:

$$y_{k+1} = y_k + hf(y_{k+1}, t_{k+1}), \tag{13}$$

where

$y_k = [c(t_k, z), q(t_k, z)]$ is the state vector,
$k$ denotes the current iteration, and
$h$ denotes the discrete timestep size.

The equations are collected into a single sparse matrix and solved with LU-factorization.

Probabilistic algorithm

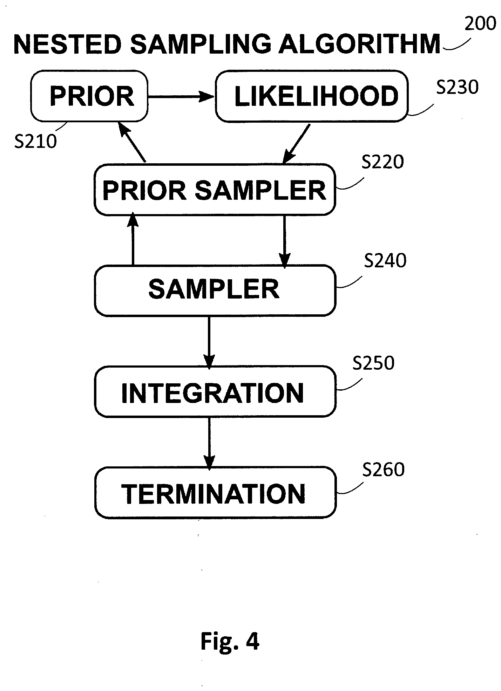

The example uses a nested sampling algorithm for Bayesian inference, such as shown in Figure 4.
The algorithm is run with $N_p$ = 400 Live points until convergence which is reached when rate of change of log Z reaches 0.5. The posterior distributions are assumed to be normally distributed implying the prior distributions can be sampled uniformly from the indicated ranges (parameter distributions) for the parameters a, $\varepsilon_c$, $k_m$, $D_L$ in **Table** 1.

From the assumption it follows that the likelihood is given by:

$$\chi^2 = \sum_i \frac{(y_i - \tilde{y}_i)^2}{\sigma} \qquad (14)$$

and

log-likelihood by:

$$\log L = \frac{\chi^2}{2}. \qquad (15)$$

**Figure** 4 shows a schematic overview of an exemplary probabilistic algorithm 200 using a nested sampling algorithm. The algorithm 200 includes providing (step S210) a prior (i.e. the prior parameter distribution), sampling (step S220) the prior with a set of points using a prior sampler and calculating the likelihood (step S230). The prior sampler employs one or more of the evaluators described above. Constraint is defined on the likelihood by the most recent removed point. At each iteration, a point may be replaced by a new one if it exceeds previous likelihood (step S240). The removed point is passed to an integrator, which weighs these dead points to form a posterior sample, and gives the marginal likelihood Z (step S250). The nested sampling algorithm terminates at step 260.

In particular, the algorithm may include the following steps:

a. Sample $N_p$ points $\theta_i, \dots, \theta_{Np}$, drawn from the prior parameter distributions, each $\theta_i$, containing one of each [a, $\varepsilon_c$, $k_m$, $D_L$]. Exemplary prior parameter distributions are shown in Table 1.

b. Iterate i = 1, ...,$N_s$ steps:

i. Calculate the smallest likelihood value out of all points $L_{min,i} = min(\{L_0, ..., L_{Np}\})$. Here the likelihood function describes the joint probability of observed data (e.g. experimental data) as a function of the parameters of the currently chosen statistical model, i.e. the currently chosen estimator. For numerical reasons the log-likelihood may be is used.

ii. Select method step, which may include a decision whether to continue to use the current evaluation method or to switch to a new method, wherein the current evaluation method may be an evaluation method realized by using one of the estimators (grid, trained PINN model or simulator). The determination whether to switch from a current evaluation method to another evaluation method (i.e. to another estimator) may be based on the following criteria that may be seen as a transition of states in the following order:

1. If current evaluation method is grid and no point with lower likelihood can be found, switch method to PINN model;

2. If current method is PINN model, check every $N_u$=20 steps residual between PINN model and simulator loglikelihood. If the residual exceeds a predetermined tolerance threshold (e.g. $e_{method}$ = 1.0), switch to simulator.

iii. If all points represent a volume in parameter space, then the volume can be shrunk by removing points with the lowest likelihood. The factor may be given by: $X_i = exp(-i/N_p)$

iv. Weights are then calculated by: $w_i = X_{i-1} - X_i$

v. Update marginal likelihood $Z$ as a product of weights and minimum likelihood: $Z = Z + L_{min,i} \cdot w_i$ When $\Delta Z$ is sufficiently small (i.e. does not exceed a predetermined threshold), return $Z$. Here $\Delta Z = Z_{i-1} - Z_i$ Here $\Delta Z$ denotes the difference in Z compared to the previous iteration.

PINN model

A PINN model employing a neural network is created according to the process described above and presented in the work (see e.g. Söderström, 2022). The neural network is trained using the learn solution approach parametrization on the $\zeta = $ [a, $\varepsilon_c$, $k_m$, $D_L$]. The different combinations of parameterizations are generated through LHS with the bounds specified in **Table 1.**

Test data is generated by saving full simulated solutions over the entire solution domain for 50 new combinations of parameterizations. These combinations of parameterizations are also generated through LHS.

Grid storage (Data storage)

Finally, an in-memory synthetic dataset containing the simulated solutions obtained by the simulator is generated from a hypercube for parameters $\theta = [a, \varepsilon_c, k_m, D_L]$. To generate the in-memory synthetic data set, the parameter boundaries in Table 1 were used with 30 points per parameter giving a total of $30^4$ *entries.* The data is loaded into a k-d tree to allow querying from the model with an average and worst-case time complexity of $O(\log n)$ and $O(n)$ respectively. When a parameter vector is queried, $\theta \to (\boldsymbol{x}, \boldsymbol{t})$, the closest matching neighbor is returned.

The search in the multidimensional data storage (grid storage) may be a nearest neighbor search. Within the context of the present disclosure, the nearest neighbor search includes any similarity search algorithm that "looks for" (i.e., searches for) entries "close" to a query. This is commonly achieved by some vector distance metric/criterium or another suitable distance metric and criterium. It is also possible to combine different search algorithms. For example, the data store (grid store) may be realized as a set of "vector" databases, which can have backends that combine various search techniques (see for example https://www.pinecone.io/learn/vector-database/). Other options may include various "spatial data partitioning trees" to which a k-d tree search algorithm belongs to, such as R-tree, M-tree. etc.

In the following, results from tests on the performance of each of the PINN model, the grid storage and the simulator are presented.

Initially the performance of each evaluator alone was measured in terms of calculation speed and error against a high-resolution simulation. Then mock setup, parameter estimation and prediction phases are presented for two synthetic experiments with two different parameter sets.

Test results

Performance benchmark

Initially a benchmark was performed to evaluate the error and speed capabilities of the different evaluation methods. To perform the benchmark 10000 parameter sets were drawn randomly from the parameter space defined in Table 1, $\theta = [a, \varepsilon_c, k_m, D_L]$. The parameter sets were then either queried from the k-d tree (i.e., from the in-memory grid storage) to determine respective solutions or evaluated with either simulator using $h = 10^{-1}$ or by PINN. Errors were calculated against simulation evaluations with $h = 10^{-2}$ as the source of truth. Evaluation times are presented in **Table 2** and errors in **Table 3**.

In particular, **Table 1** shows an exemplary parameter space defined for training of PINN model and for sampled parameters in benchmarks and synthetic experiments.

**Table 1**

| Simulation parameters | Values / Value ranges |
|---|---|
| Column length L | $L = 1\ m$ |
| Maximum evaluation time $t_{max}$ | $t_{max} = 100\ S$ |
| Axial dispersion coefficient $D_L$ | $D_L \in [10^{-9}, 10^{-2}]\ m^2 s^{-1}$ |
| External porosity $\varepsilon_c$ | $\varepsilon_c \in [0.3, 0.7]$ |
| Lumped mass transfer coefficient $k_m$ | $k_m \in [10^{-5}, 1]\ s^{-1}$ |
| Henry coefficient $a$ | $a \in [10^{-5}, 1]\ s^{-1}$ |
| Interstitial velocity $u$ | $u = 0.1\ ms^{-1}$ |
| Injection concentration $c_{inj}$ | $c_{inj} = 1\ gl^{-1}$ |
| Injection time $t_{inj}$ | $t_{inj} = 100\ s$ |

**Table 2** shows evaluation time benchmarks (evaluation time results) for the different methods. 10 000 evaluations were performed for each method showcasing the difference in evaluation time. The final column indicates the time it takes to load the model into memory, and is only applicable for the PINN model and k-d tree and only performed once before the benchmark is started.

**Table 2**

| Method | Total time | Single time | Model load time |
|---|---|---|---|
| PINN | 17.01 s | 0.001701 s | 4.7 s |
| k-d tree | 1.190 s | 0.0001190 s | 172.5 s |
| simulation $h = 10^{-1}$ | 49.55 s | 0.004955 s | N/A |

(continued)

| Method | Total time | Single time | Model load time |
|---|---|---|---|
| simulation h = $10^{-2}$ | 478.4 s | 0.04784 s | N/A |

As seen from Table 2, there is a significant reduction of the evaluation time required by the PINN model and the k-d tree, as compared to a numerical simulation.

**Table 3** shows resulting evaluation error benchmarks for the various methods. Again, 10 000 evaluations were performed for each benchmark.

**Table 3**

| | k-d tree | PINN | simulation $h$ = $10^{-1}$ |
|---|---|---|---|
| mean [$g^2/l^2$]<br>std [$g^2/l^2$] | $7.06 \cdot 10^{-5}$<br>$6.17 \cdot 10^{-4}$ | $1.34 \cdot 10^{-4}$<br>$2.06 \cdot 10^{-4}$ | $1.076555 \cdot 10^{-5}$<br>$2.911855 \cdot 10^{-5}$ |
| min [$g^2/l^2$]<br>max [$g^2/l^2$] | $1.71 \cdot 10^{-10}$<br>$2.40 \cdot 10^{-2}$ | $0.1 \cdot 10^{-5}$<br>$4.294 \cdot 10^{-3}$ | $4.708937 \cdot 10^{-7}$<br>$6.466970 \cdot 10^{-4}$ |

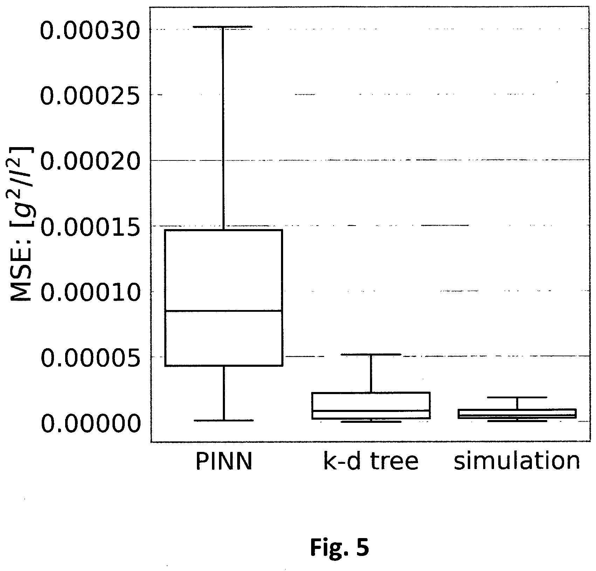

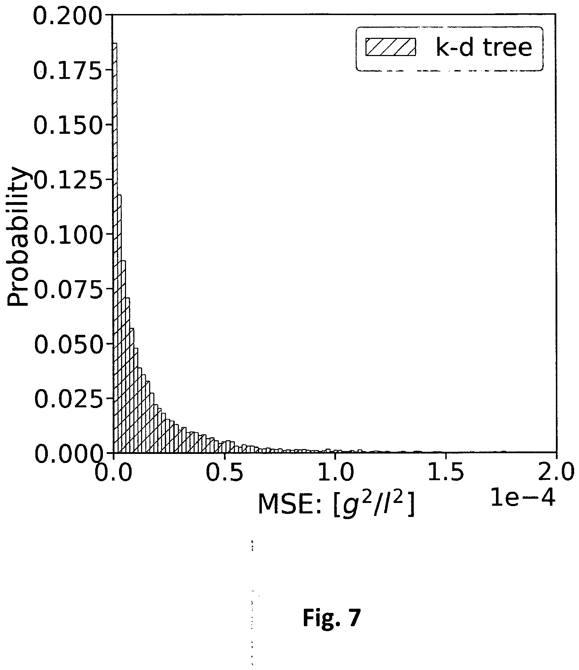

The results of the benchmark tests are shown in **Figures 5** to **9,** wherein **Figure 5** shows a boxplot visualizing the errors of each method and **Figures 7** to **10** show histograms of error distributions.

In particular, **Figure 5** shows the MSE error for the above described initial benchmark runs for each method (i.e. simulator, grind and PINN), where errors are calculated using high resolution simulation (i.e. simulation with $h = 10^{-2}$) as truth. 10000 evaluations were performed using the same parameter set.

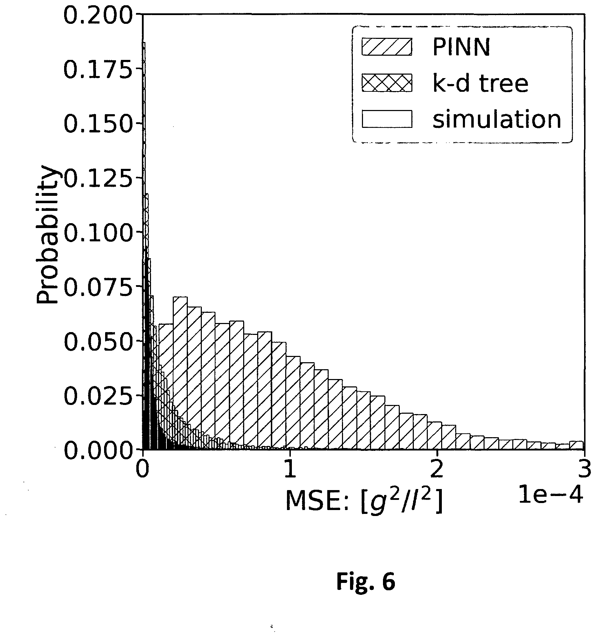

**Figure 6** shows normalized histograms of the MSE errors in units of [$g^2/l^2$] (horizontal axis) and the probability (vertical axis) of the evaluation methods in the initial benchmark for each method (i.e. for k-d tree queries of the grid data storage, PINN model and simulation).

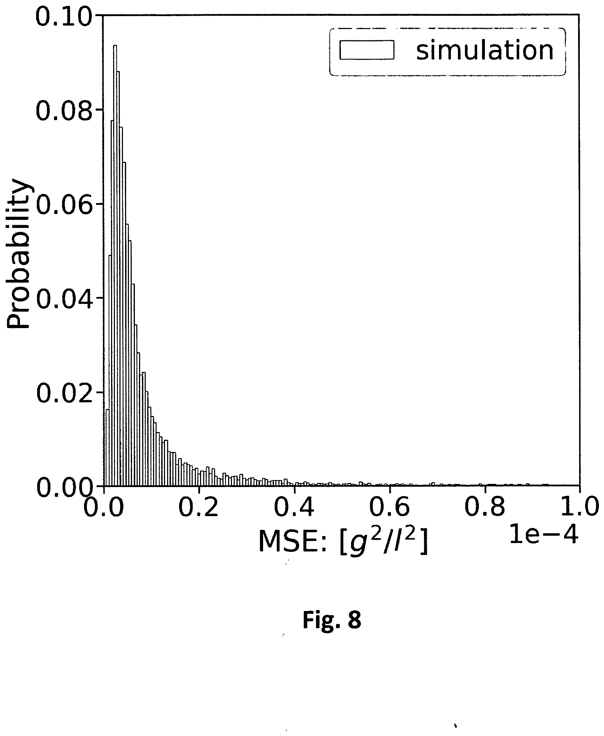

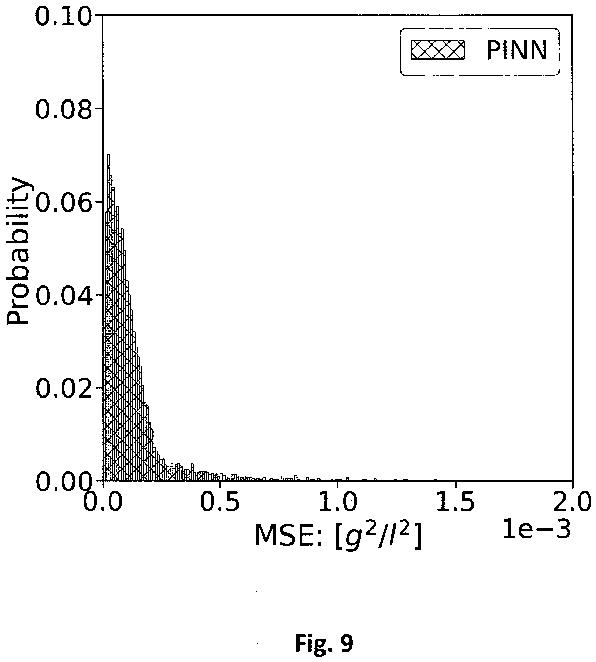

**Figures 7** to **9** show the respective error distribution for each of the k-d tree queries of the grid data storage (Figure 7), the simulator evaluations (Figure 8) and the PINN evaluations (Figure 9) in the initial benchmark.

Case studies using synthetic experimental data

Case studies were performed by drawing a random parameter set from the space defined in Table 1. A simulation using the randomized set was performed and the vector of the eluate curve saved. Subsequently a synthetic experimental dataset is generated from the curve by drawing random samples. The sampling is done by picking [50,100] points on the curve and using it as the mean in a normal distribution, $X = N(\mu,\sigma^2)$. The new vector of synthetic experimental data is designated $\tilde{y}$ as in Figure 4. A value of $\sigma = 0.025$ was used.

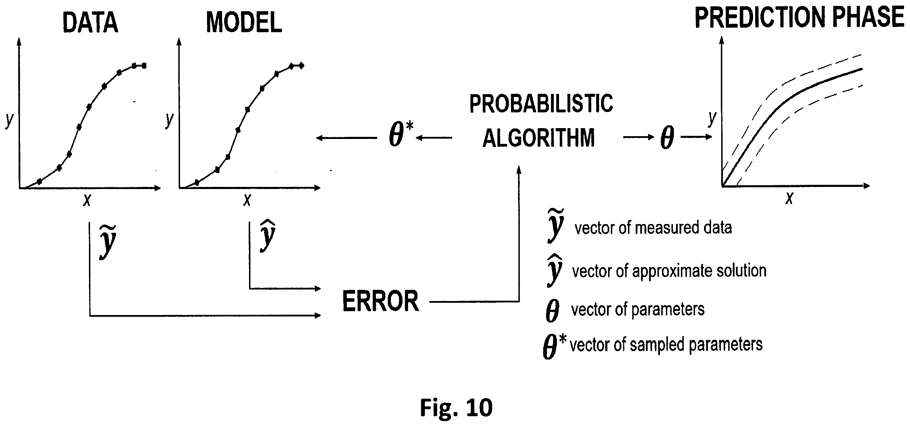

**Figure 10** shows an overview of stages of an exemplary method for configuring and/or controlling a chromatography method and/or system, for example as used in an application phase. As shown from the left to the right of Figure 10, input data and model data may be used to optimize parameters. An exemplary probabilistic algorithm, such as the one described above, generates a-posterior distributions for sought parameters in the physical model and the respective credible intervals which are then used in a prediction phase.

To summarize the procedure, in the method shown in Fig. 10, a curve is simulated from random parameters (referred to as the "true" parameters), points are sampled from the curve and noise added to generate synthetic experimental data. The objective is then to recover the true parameters using a probabilistic method as described above in connection with Figures 1 to 4. The experiment is repeated twice to show variations in performance. The two repetitions are referred to as "case 1" and "case 2".

Evaluation times for a completed parameter estimation phase for cases 1 and 2 are shown **Table 4.** As shown in Table 4, the k-d tree and PINN are significantly faster than the simulator.

**Table 4**

| Method | Total evaluation time case 1 | Total evaluation time case 2 |
|---|---|---|
| PINN<br>k-d tree<br>simulation $h$ = $10^{-1}$ | 146.98 s<br>34.3 s<br>2040.32 s | 155.11s<br>31.21 s<br>1917.0 s |

**Tables 5** and **6** show the approximations obtained using each method and the true parameter values, wherein **Table 5** shows the final parameter approximations after application stage termination with true parameters for reference for case 1 and **Table 6** shows the final parameter approximations after application stage termination with true parameters for reference for case 2. The methods all manage to place the true value within the confidence/credibility boundaries except for the PINN failing to find the correct axial dispersion in case 2.

**Table 5**

| Parameter | True | k-d tree | PINN | simulation $h$ = 10⁻¹ |
|---|---|---|---|---|
| $\varepsilon_c$ | 0.4261 | 0.438 $\pm$ 0.094 | 0.424 | 0.437 $\pm$ 0.077 |
| $D_L$ | 0.0073 | 0.0067 | $\pm$ 0.074 | 0.0064 $\pm$ 0.0011 |
| $k_m$ | 0.0904 | $\pm$ 0.0011 | 0.0072 | 0.087 $\pm$ 0.014 |
| $a$ | 0.5506 | 0.087 $\pm$ 0.021 | $\pm$ 0.013 | 0.61 $\pm$ 0.19 |
| | | 0.63 $\pm$ 0.24 | 0.098 | |
| | | | $\pm$ 0.016 | |
| | | | 0.57 $\pm$ 0.19 | |

**Table 6**

| Parameter | True | k-d tree | PINN | simulation $h$ = 10⁻¹ |
|---|---|---|---|---|
| $\varepsilon_c$ | 0.511 | 0.53 $\pm$ 0.11 | 0.48 $\pm$ 0.12 | 0.51 $\pm$ 0.11 |
| $D_L$ | 9.6 | (7.3 $\pm$ 3.0) | (0.4 $\pm$ 1.1) $\cdot$ 10⁻⁴ | (1.5 $\pm$ 2.2) $\cdot$ 10⁻⁴ |
| $k_m$ | $\cdot$ 10⁻⁴ | $\cdot$ 10⁻⁴ | 0.64 $\pm$ 0.16 | 0.56 $\pm$ 0.16 |
| $a$ | 0.822 | 0.67 $\pm$ 0.18 | 0.29 $\pm$ 0.13 | 0.30 $\pm$ 0.14 |
| | 0.259 | 0.32 $\pm$ 0.13 | | |

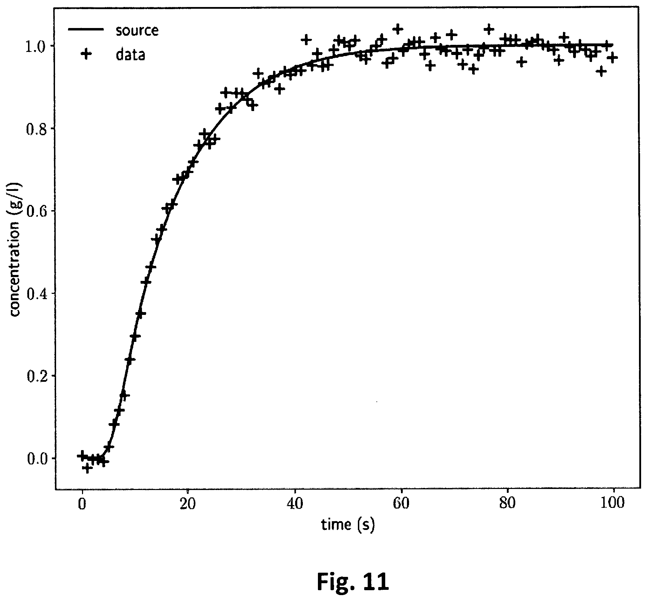

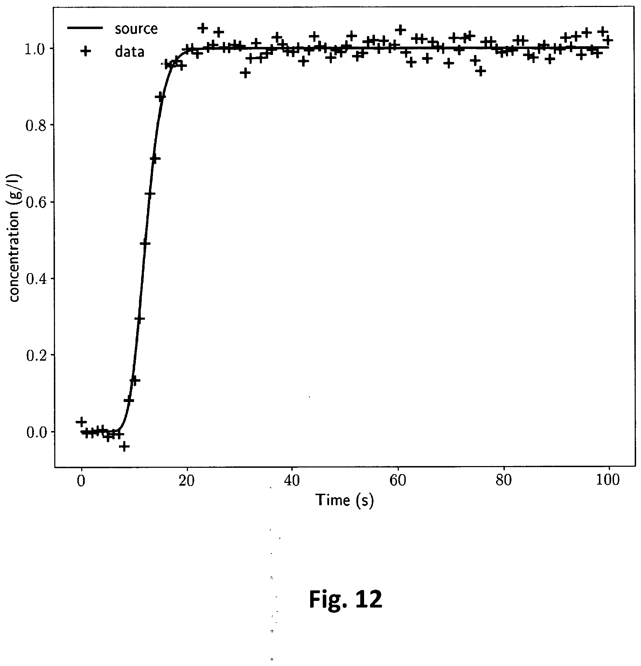

**Figures 11** and **12** show the synthetic experiment data and the respective curve from which they are sampled, wherein **Figure 11** shows synthetic experimental data and source curve simulated from true parameters for case 1 and **Figure 12** shows synthetic experimental data and source curve simulated from true parameters for case 2. Specifically, Figures 11 and 12 show the concentration in [g/l²] (vertical axis) as a function of the time in [s] (horizontal axis). In Figures 11 and 12 the source data is indicated as continuous line and the sampled synthetic experimental data points are indicated by "+" sign.

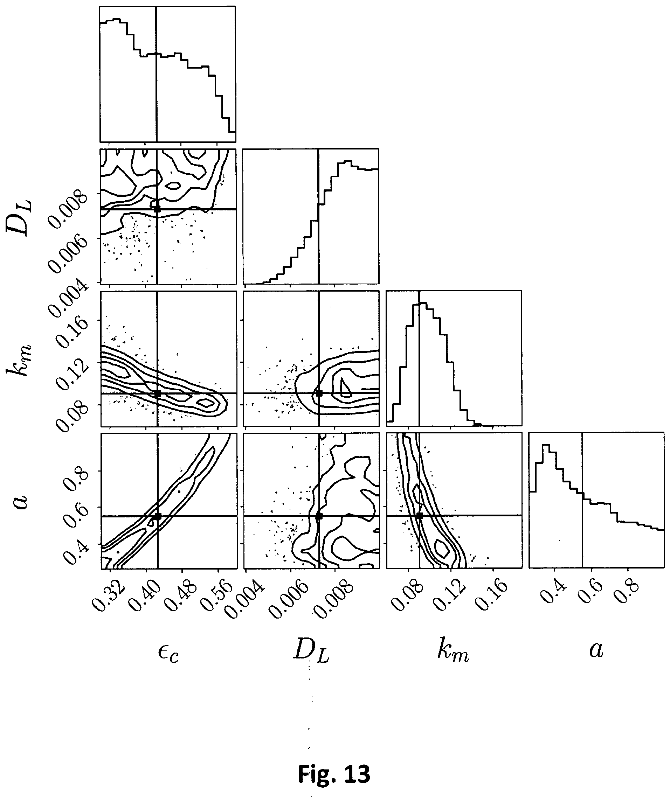

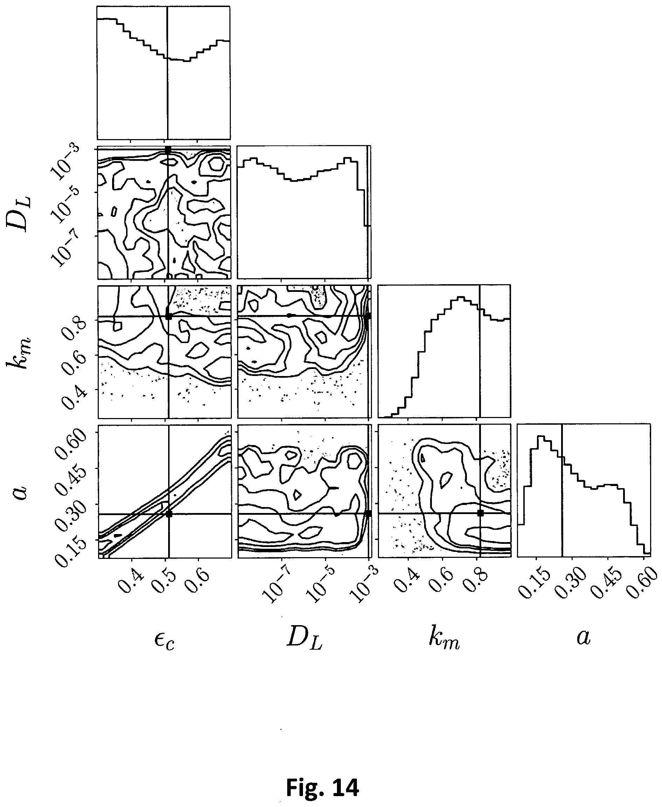

**Figures 13** and **14** show the posterior distributions for the parameters *"$D_L$", "a", "$k_m$"* and "$\varepsilon_c$" shown in Tables 5 and 6. The posterior distributions are generated using the PINN model alone for case 1 and 2 respectively. In particular, **Figure 13** shows posterior parameter distributions generated using PINN model with true parameters marked for case 1 and **Figure 14** shows posterior parameter distributions generated using PINN with true parameters marked for case 2.

Figure 13 shows a flat distribution for dispersion $D_L$ indicating the model fails to inform us about the distribution of that parameter in this case.

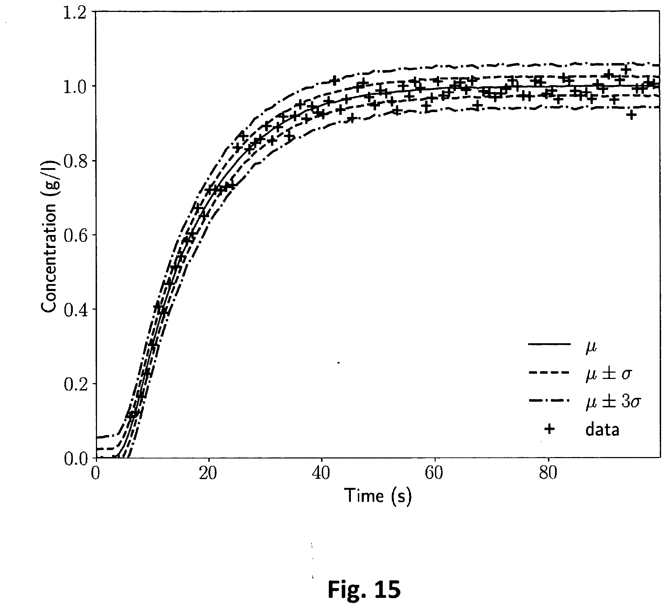

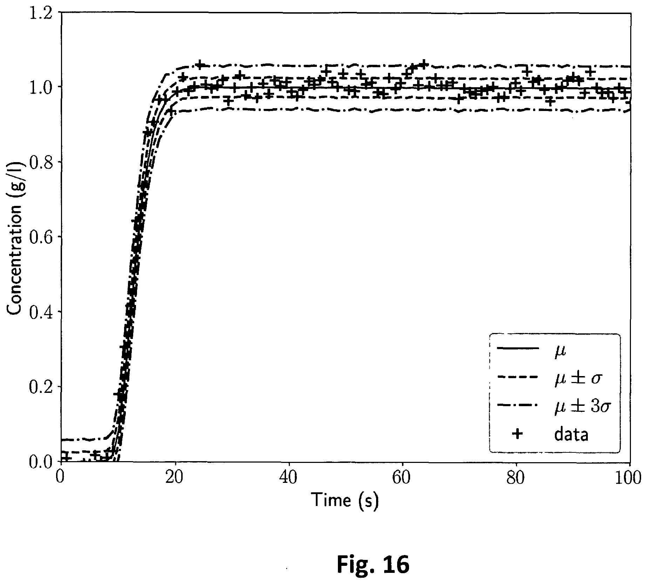

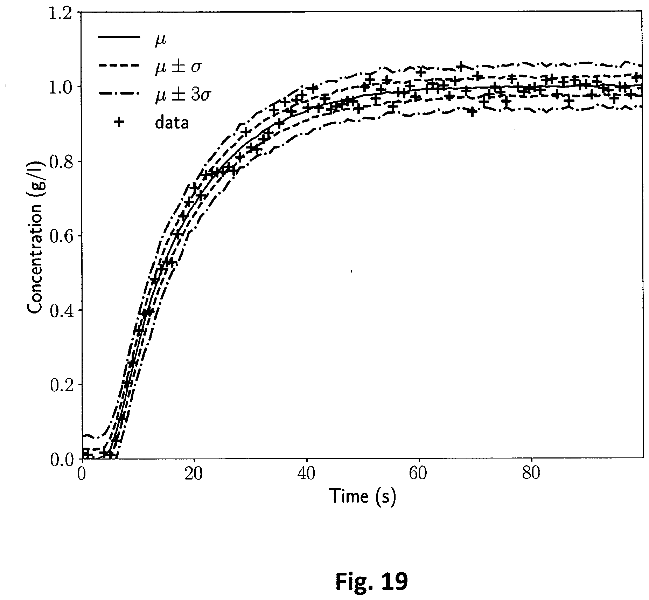

**Figures 15** and **16** show the distribution of predicted concentrations in [g/l] as a function of time derived from the generated posterior parameter distributions. **Figure 15** shows synthetic experimental data (indicated as "+") overlayed with credible intervals generated using posterior probability derived from PINN for case 1. **Figure 16** shows synthetic experimental data (indicated as "+") overlayed with credible intervals generated using posterior probability derived from PINN for case 2. Figures 15 and 16 show further the true (source) curve. As seen from Figures 15 and 16, even though the PINN failed to find the correct dispersion value, it correctly centers the true curve within its confidence/credibility bounds. In Figures 15 and 16, the boundaries of the respective credible intervals indicate that the probability of a prediction sampled from the posterior falling within the respective boundary is 68% (see line "$\mu \pm \sigma$") and 99.7% (see line "$\mu \pm 3\sigma$") from the mean $\mu$. This also applies to all other figures (see Figures 19, 20, 23 to 26 and 32) showing credible intervals. For the sake of clarity, it is noted that a *sample* here refers to a single vector of parameters = [, , ,] of the list of posterior samples stored by the algorithm.

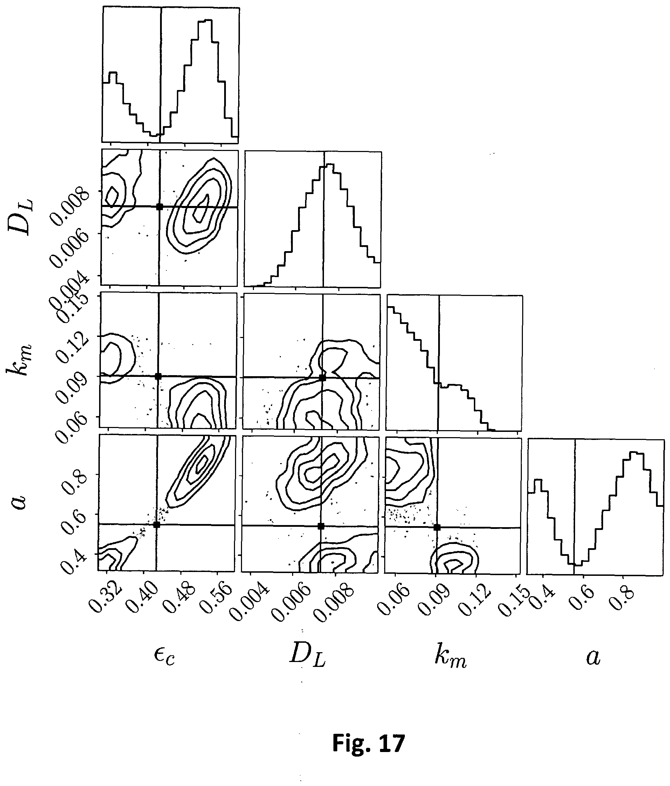

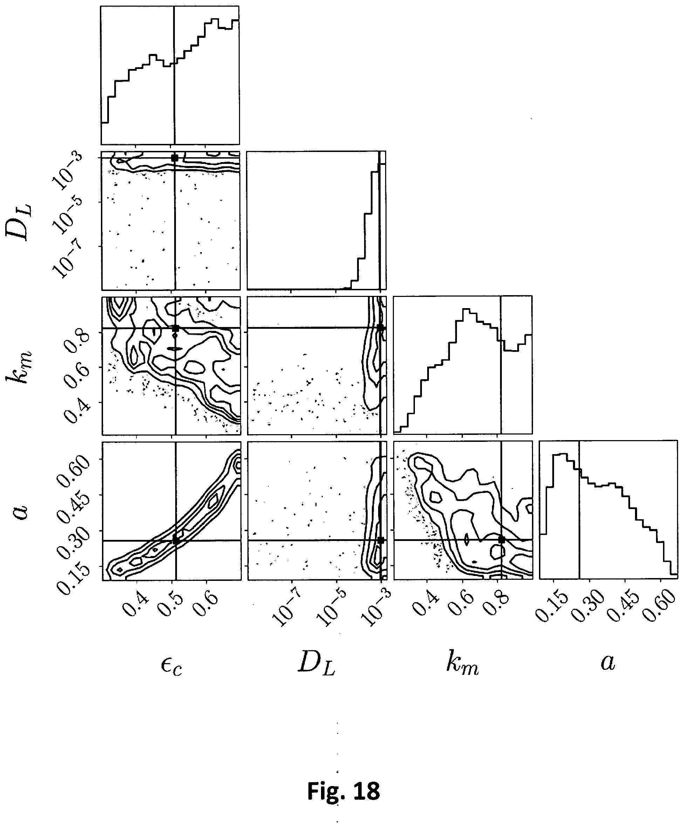

**Figures 17** and **18** show posterior distributions for the parameters *"$D_L$", "a", "$k_m$"* and "$\varepsilon_c$" shown in Tables 5 and 6 generated using the k-d tree for case 1 and 2 respectively **Figure 17** shows the posterior parameter distributions generated using k-d tree with true parameters marked for case 1 and **Figure 18** shows the posterior parameter distributions generated using k-d tree with true parameters marked for case 2.

As seen from Figure 17 there are splits in the distributions of porosity and adsorption constant. This is an artefact due to the discretization of the grid creating an artificial divide in-between the grid steps.

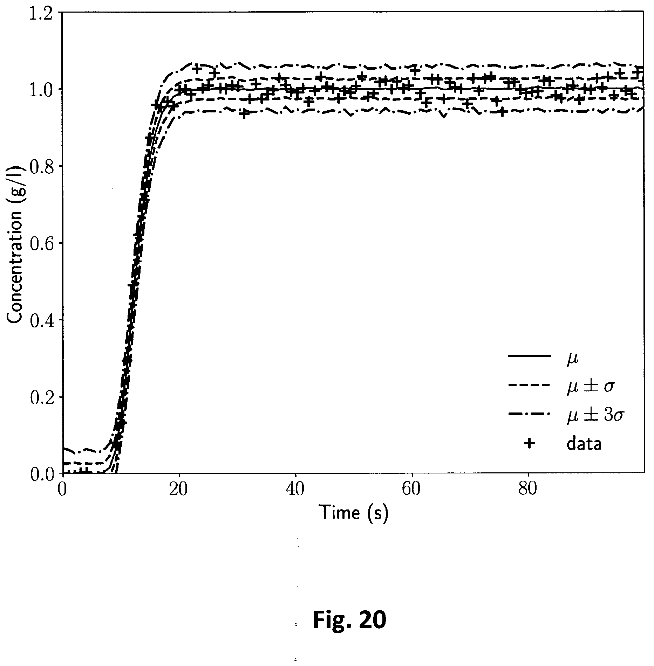

**Figures 19** and **20** shows the predicted concentration distribution derived from the generated posterior parameter distributions, wherein **Figure 19** shows the synthetic experiment data overlayed with credible intervals ($\mu \pm 3\sigma$ and $\mu \pm \sigma$) generated using posterior probability derived from k-d tree for case 1 and **Figure 20** shows synthetic experiment data overlayed with credible intervals generated using posterior probability derived from k-d tree for case 2. Further, Figures 19 and 20 show the true (source) curve overlayed on the experimental data (indicated as "+"). As seen from Figures 19 and 20, the k-d tree prediction has correctly centered the true curve within its confidence/credibility bounds despite the split distributions seen in Figure 17.

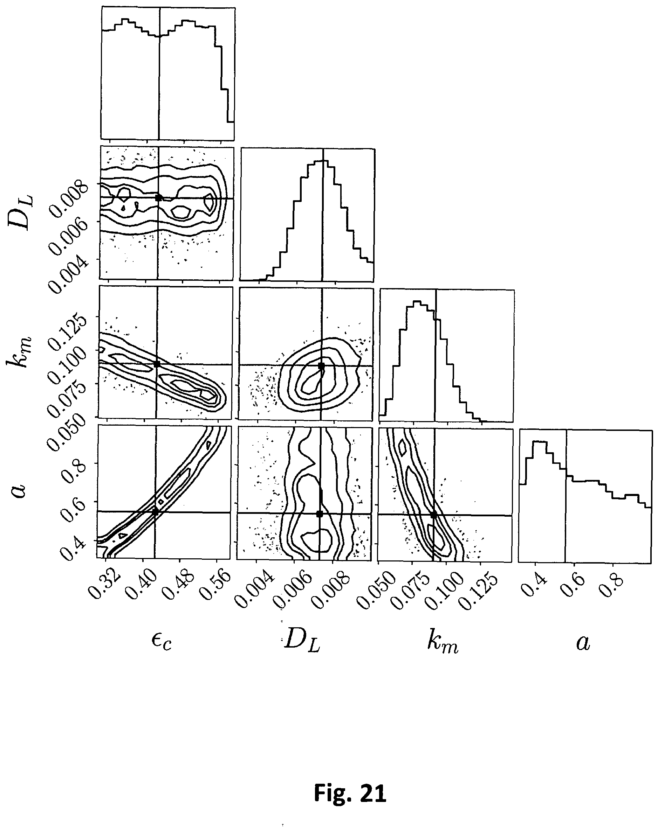

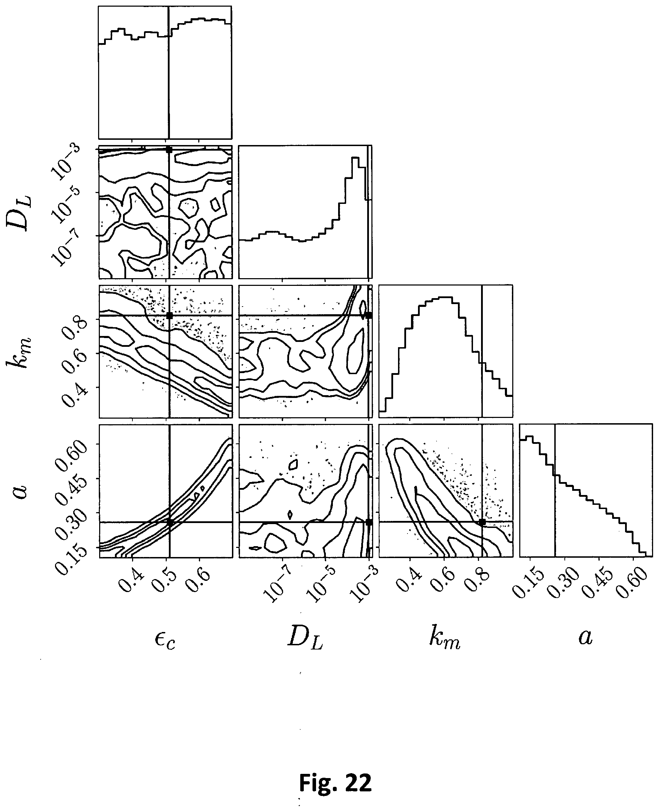

**Figures 21** and **22** show the posterior distributions generated using the simulator for case 1 and 2 respectively, wherein **Figure 21** shows parameter distributions generated using simulator with true parameters marked for case 1 and **Figure 22** shows parameter distributions generated using simulator with true parameters marked for case 2. As seen from Figures 21 and 22, adsorption and porosity show flat distributions and dispersion and mass transfer coefficients more defined distributions.

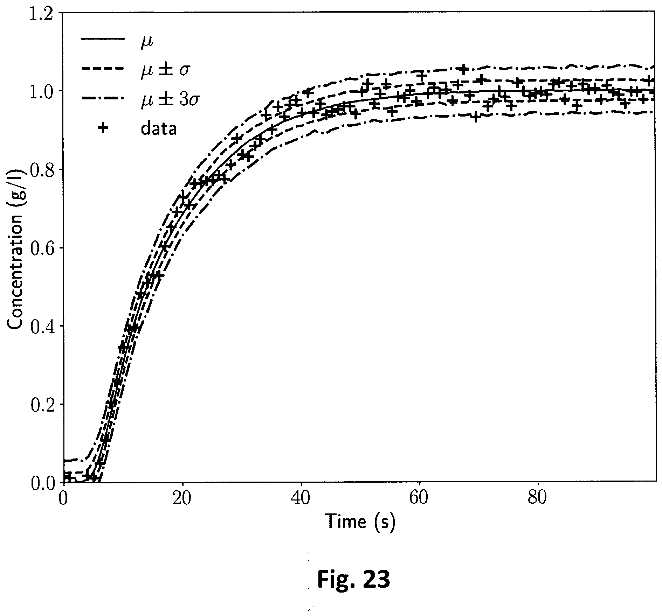

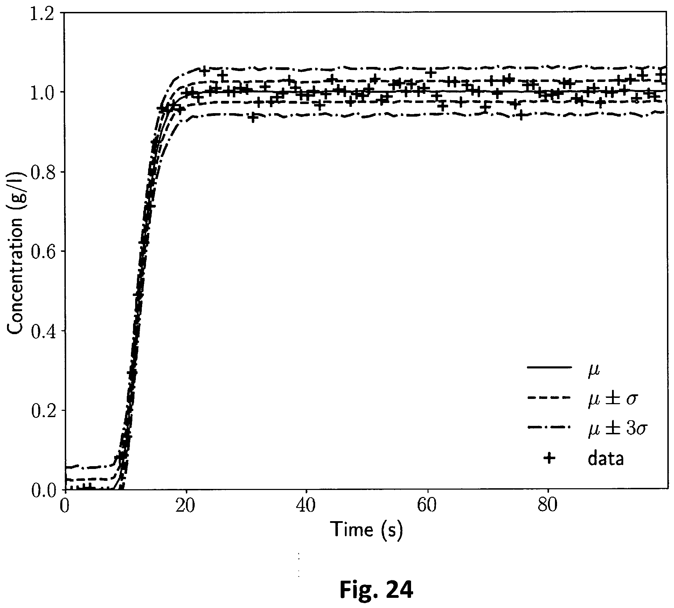

**Figures 23** and **24** show the predicted concentration distribution derived from the posterior parameter distributions, wherein **Figure 23** shows synthetic experiment data overlayed with credible intervals ($\mu \pm 3\sigma$ and $\mu \pm \sigma$) generated using posterior probability derived from simulation for case 1 and **Figure 24** shows synthetic experiment data overlayed with credible intervals ($\mu \pm 3\sigma$ and $\mu \pm \sigma$) generated using posterior probability derived from simulation for case 2. The experimental data and true curve are shown overlayed in Figures 23 and 24. As seen from Figures 23 and 24, the estimation correctly centers the true curve within its confidence/credibility bounds.

A comparison of the results shown in Figures 13 to 20 with those shown in Figures 21 to 24, which are obtained by a high-resolution simulation, confirms that the PINN evaluation and the grid evaluation may be used to a great extend before switching to the simulator. For example, the PINN may be used to "carve" off a lot of samples before switching method, since the distributions show a similar shape and constrain the boundaries of each individual parameter.

Case study using experimental data

An additional case study was performed based on experimental data of a 5ml affinity chromatography breakthrough curve obtained by measuring absorbance at 280nm.

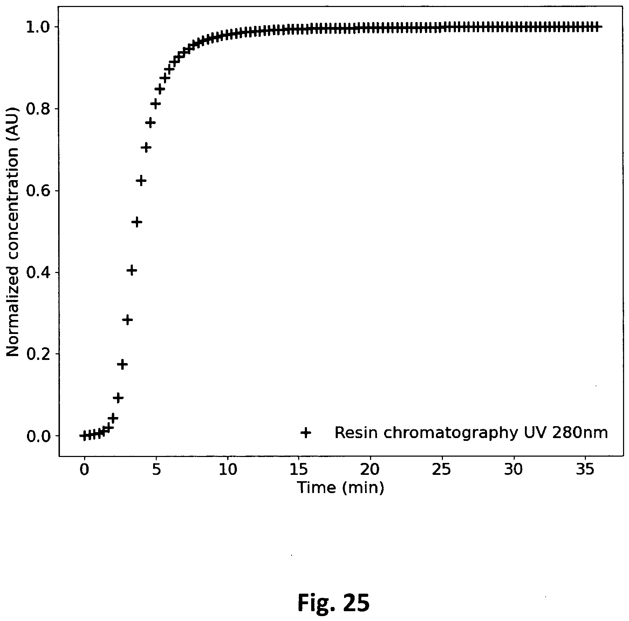

The experimental data is visualized in **Figure 25,** which shows the obtained normalized concentration in units [AU] as a function of the time. A constant measurement error value of $\sigma = 0.02$ was used and the intensity of the spectrum normalized with the measured maximum. The sought parameters were $\theta = [a, \varepsilon_c, k_m, D_L]$. The prior distributions used are defined in **Table 7** together with physical experiment parameters for the experimental case sturdy.

**Table 7**

| Experimental parameters | Values |
|---|---|
| Column length $L$ | $L = 2.5$ *cm* |
| Maximum time $t_{max}$ | $t_{max} = 37$ min |
| Axial dispersion coefficient $D_L$ | $D_L \in [10^{-9}, 10^{-2}]$ cm$^2$min$^{-1}$ |
| External porosity $\varepsilon_c$ | $\varepsilon_c \in [0.3, 0.7]$ |
| Lumped mass transfer coefficient $k_m$ | $k_m \in [10^{-5}, 1]$ min$^{-1}$ |
| Henry coefficient $a$ | $a \in [10^{-5}, 1]$ min$^{-1}$ |
| Interstitial velocity $u$ | $u = 1.4$ *ml* $\cdot$ min$^{-1}$ |
| Injection time $t_{inj}$ | $t_{inj} = 37$ min |

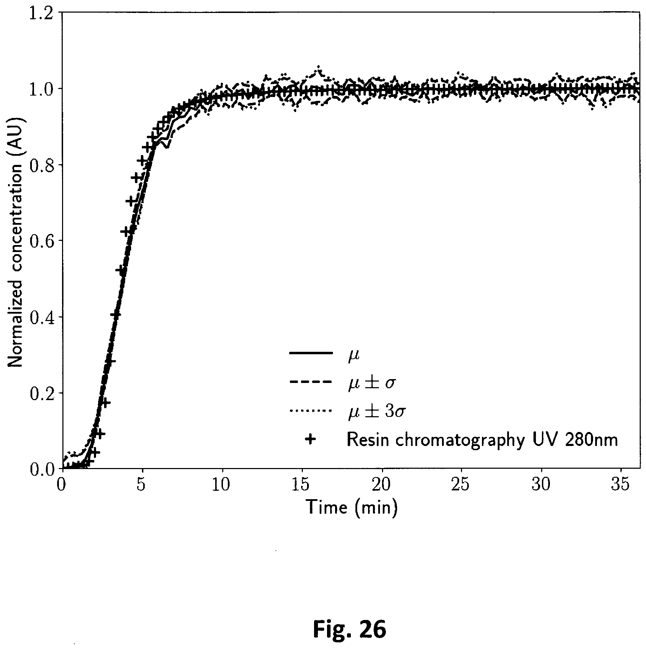

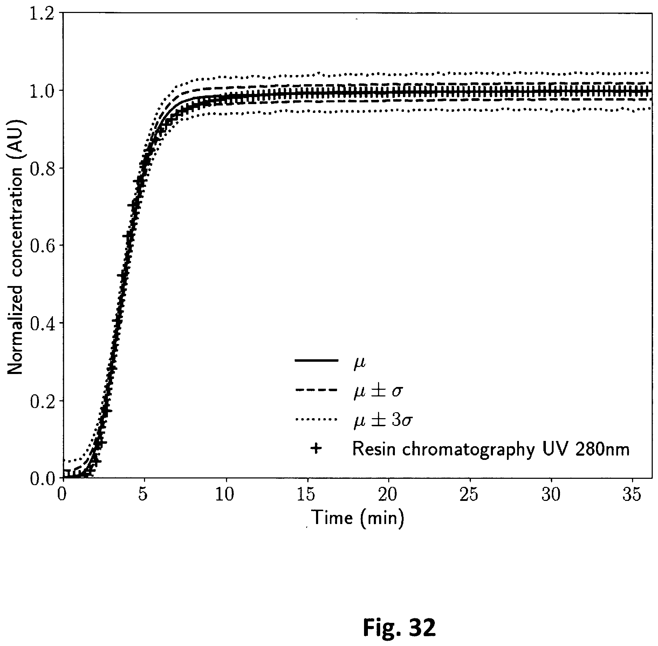

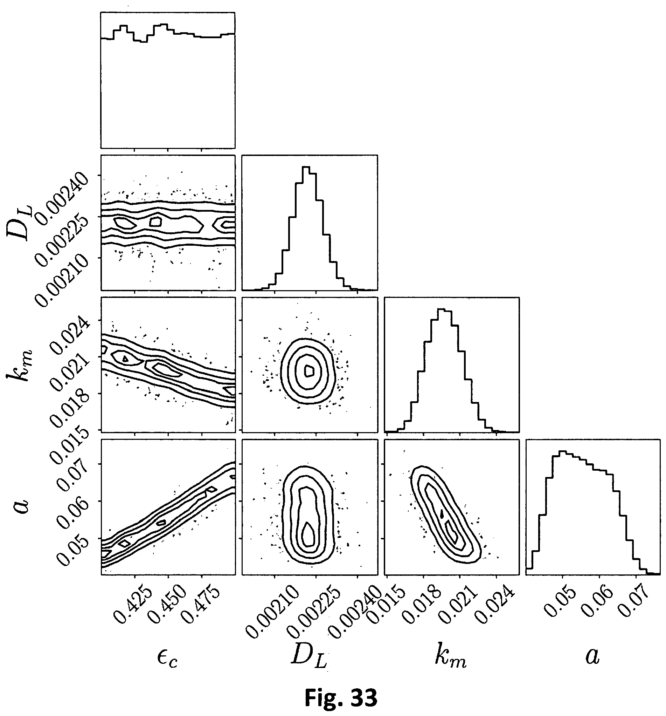

For reference, individual evaluations of each method are provided in **Tables 8** to **10,** and **Figures 26** to **31.** Then a *combined mode* where there is a transition between all three methods in the order grid, PINN, simulator as described above, for example in connection with the algorithm described in connection with Figures 1 to 4, was carried out. The results from *combined mode* are shown in **Table 11** and **Figures 32** and **33.**

In particular, **Table 8** shows posterior parameter means and single standard deviation limits from grid alone.

**Table 8**

| Property | Value | Error [+/-] |
|---|---|---|
| logZ | -444.3 | 1.118 |
| Time | 52.2s | 0.0 |
| $\varepsilon_c$ | 0.478 | 0.0039 |
| $D_L$ | 0.005158 | 0.000093 |

(continued)

| Property | Value | Error [+/-] |
|---|---|---|
| $k_m$ | 0.0654 | 0.0096 |
| a | 0.1056 | 0.0061 |

**Table 9** shows posterior parameter means and single standard deviation limits from PINN alone.

**Table 9**

| Property | Value | Error [+/-] |
|---|---|---|
| logZ | -119.98 | 0.203 |
| time | 458.3s | |
| $\varepsilon_c$ | 0.417 | 0.019 |
| $DL$ | 0.004012 | 0.000047 |
| $k_m$ | 0.1003 | 0.0041 |
| $a$ | 0.010 | 0.017 |

**Table 10** shows posterior parameter means and single standard deviation limits from simulator:

**Table 10**

| Property | Value | Error [+/-] |
|---|---|---|
| logZ | -2444.4 | 0.194 |
| time | 790.2s | 0.0 |
| $\varepsilon_c$ | 0.50 | 0.12 |
| $D_L$ | 0.002223 | 0.000045 |
| $k_m$ | 0.0181 | 0.0042 |
| $a$ | 0.075 | 0.035 |

**Table 11** shows posterior parameter means and single standard deviation limits using the combined algorithm using all three methods (i.e. using combined grid (k-d tree), PINN and simulator), wherein each stage time is indicated.

**Table 11**

| Property | Value | Error [+/-] |
|---|---|---|
| logZ | -2439.1 | 0.150 |
| time | 20.1 + 91.68 + 474.14 = 585.92s | 0.0 |
| $\varepsilon_c$ | 0.449 | 0.029 |
| $D_L$ | 0.002224 | 0.000045 |
| $k_m$ | 0.0198 | 0.0013 |
| $a$ | 0.075 | 0.0555 |

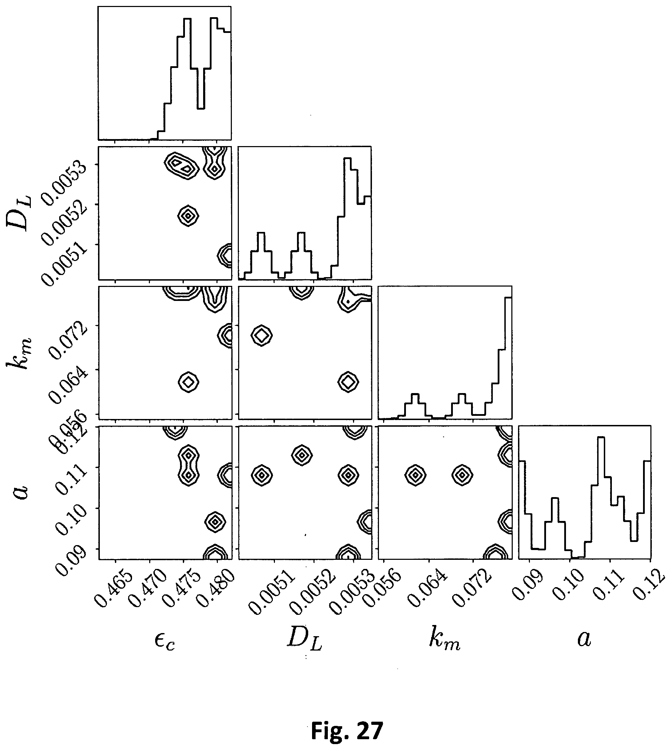

**Figures 26, 28, 30** and **32** show experimental data (shown as continuous line) overlayed with credible intervals generated using posterior probability generated by a probabilistic algorithm using a single one of the evaluation methods (i.e. k-d tree, PINN or simulator) or a combination of all three evaluation method according to an exemplary method described herein. Specifically, the continuous line in each of Figures 26, 28, 29 and 32 shows the measured normalized concentration in arbitrary units (AU) as a function of the time in [min] of a reason chromatography. The concentration is measured by 280 nm UV spectrography. **Figures** 27, **29, 31** and **33** show the respective posterior parameter distributions generated by a posterior probability algorithm employing a single one of the evaluation methods (i.e. k-d tree, PINN or simulator) or a combination of all three evaluation method according to an exemplary method described herein.

In particular, **Figure 26** shows experimental data overlaid with credible intervals ($\mu \pm 3\sigma$ and $\mu \pm \sigma$) generated using posterior probability derived from k-d tree as evaluation method. **Figure 27** shows parameter distributions generated using k-d tree only. The grid discretization is visible in the samples.

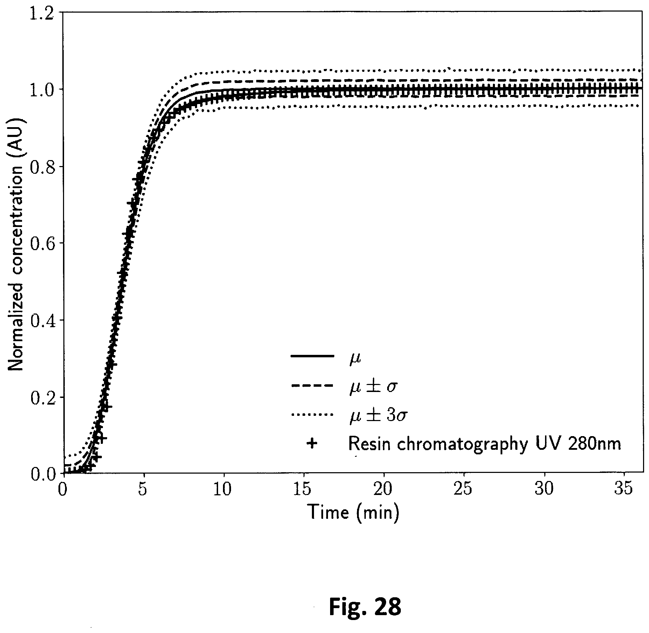

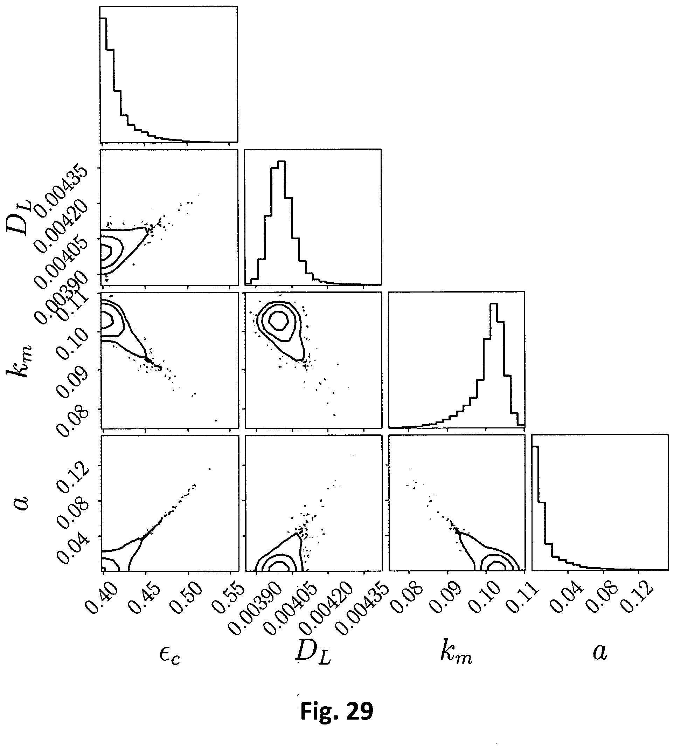

**Figure 28** shows experiment data overlaid with credible intervals ($\mu \pm 3\sigma$ and $\mu \pm \sigma$) generated using posterior probability derived from PINN as evaluation method. **Figure 29** shows parameter distributions generated using PINN only.

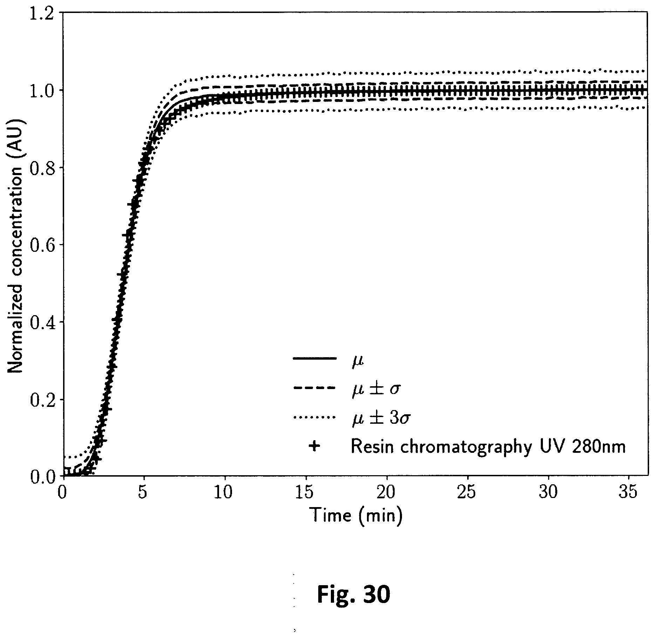

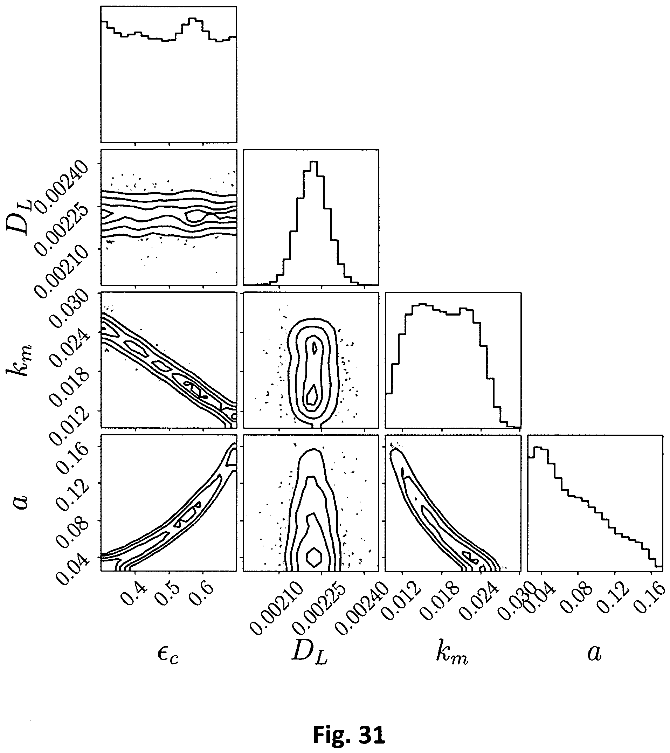

**Figure 30** shows experiment data overlaid with credible intervals ($\mu \pm 3\sigma$ and $\mu \pm \sigma$) generated using posterior probability derived from the simulator only. **Figure 31** shows parameter distributions generated using simulator only.

**Figure 32** shows experiment data overlaid with credible intervals ($\mu \pm 3\sigma$ and $\mu \pm \sigma$) generated using posterior probability derived from the combined usage of all methods (i.e., the grid, the PINN and the simulator). **Figure 33** shows parameter distributions generated using combined methods.

The experimental results shown in Figures 26 to 33 confirm that it is possible to solve the inverse problem for the experimental data in Figure 25 with a sufficient accuracy. As can be seen in Figures 26, 28 and 30, the evaluation methods by themselves create a distribution that is close to the data, but the simulator must be relied on as the source of truth. The experimental results further confirm that the combined mode achieves significantly better results that the grid and PINN alone and that the combined mode achieves comparable parameter distributions to simulator. Thus, the combined mode is capable as recreating the simulated results shown in Figure 30, but at a significantly reduced computation time. Note that the logZ values are not comparable between methods as the number of datapoints in the log-likelihood vary. Nevertheless, as seen in Tables 10 and 11 the small difference in logZ value indicate that both methods have performed equally well.

Based on the generated posterior distributions and credible intervals such as shown above, the impact of variation of one or more model parameters (such as column length, etc.) may be simulated and evaluated. Based on the results of the evaluation, the respective state parameter of the chromatography system and/or process represented by the model parameter (e.g., the column length) may be adjusted, for example in the manner described above.

As described herein, the predictive posterior distribution and/or credible intervals generated by the probabilistic algorithm may be used for various technical applications. In the following, two exemplary technical applications are described.

**Application example 1:** Control and/or configuration of a chromatography system performing a chromatography process

A method for control and/or configuration of a chromatography system performing a chromatography process may include the following steps:

a. Execute the setup phase as described above. This may enable calibration of the chromatography system and/or process.

b. Execute a parameter estimation phase based on initial experiments. The parameter estimation phase may include finding parameters and/or posterior distributions in initial experiments. At this stage, the method according to aspects, variations and examples of the invention can be used to find parameters and/or posterior distributions for different chromatography columns with varying properties to make economic predictions in or for a process stage.

c. During a chromatography process (e.g. current chromatography process), use on-line measurements (i.e. measurements of one or more physical property during the execution of the chromatography process) to predict likely outcome of process. Further, likely confidence/credibility bounds may be estimated to predict upper and lower bounds on yield, productivity and/or profitability and/or other properties. This stage may be a part of a prediction phase, which in turn may be a part process monitoring and/or control stage.

i. If on-line measurements diverge from likely boundaries supplied by posterior distribution, process operators can be signaled for action, for example by automatically generating and displaying a warning message. For example, the divergence could indicate process malfunction, column degradation or another unforeseen event requiring investigation. Thus, the signal for action (e.g. in form of a warning message) may include the detected problem(s) and an indication or proposal for the respective action (e.g. change of consumables or other process and/or system parameters)

ii. In addition or alternatively, on-line measurements can be used to predict the distribution of likely results instead of point estimates. Integrating the curve using the predictive posterior can give an approximation of total yield given the current situation with a margin of error. The yield estimate can be used to derive economical approximations and support decision making during process. For example, based on the yield estimate, a decision may be taken to change or not change at least one parameter of the chromatography process and/or system.

**Application example 2:** Control and/or configuration of a bioreactor system performing a cell line process intensification (as an exemplary bioreactor process)

In a cell line process intensification, the goal is typically to maximize output. This can for example be achieved by moving from a batch to perfusion process according to the following control and/or configuration method:

a. Execute the setup phase as described above on a bioreactor cell growth model. The setup phase may be used to calibrate the bioreactor process and/or system.

b. Execute a parameter estimation phase based on batch experiments. The parameter estimation phase may comprise finding parameters in initial experiments on a batch process.

c. Execute a prediction phase including predicting the likely output on a perfusion process by using the method according to aspects, embodiments and examples of the invention. Further, the likely total yield confidence/credibility bounds may be approximated to predict upper and lower bounds on productivity and profitability, for example. This phase may be a part process monitoring and/or control stage.

d. During a perfusion process, use on-line measurements to compare with likely outcome of process from posterior predictive distribution, for example as a part of a process monitoring and/or control stage.

i. If on-line measurements diverge from likely boundaries supplied by posterior distribution, process operators can be signaled for action, for example by generating and displaying a warning message. The signal for action may for example indicate that the cells are failing, feed media needs to be adjusted, process conditions need to be changed, or another unforeseen event. Thus, the signal for action (e.g. in form of a warning message) may include the detected problem(s) and an indication or proposal for the respective action (e.g. adjustment of feed media and/or other process and/or system parameters)

ii. In addition or alternatively, on-line measurements can be used to predict the likely result of the process, for example in terms of titre and/or yield. The titre and/or yield estimate can be used to derive economical approximations and support decision making during process. For example, based on the titre and/or yield estimate, a decision may be taken to change or not change at least one parameter of the bioreactor process and/or system.

The simulation and experimental results confirm that method for configuration and/or control of a bioprocessing system configured to perform a bioprocess according to the above aspects, variations and examples produces technical advantages over known methods. In particular, the use of a probabilistic algorithm which employs a combination of evaluation methods enables a substantial reduction of the computational time for a complete calculation of the posterior distributions, while at the same time keeping or improving the evaluation quality as compared to that of the individual evaluation method.

In addition, it is shown that the PINN model is a capable function approximator in the context of the described method and able to generate results at higher rate.

In summary, the method for configuration and/or control of a bioprocessing system configured to perform a bioprocess according to the above aspects, variations and examples may have one or more of the following properties and/or advantages:

- The PINN performance as a function approximator in the context of this problem may improve computational performance significantly.
- The approximation error from the PINN is generally not large enough to generate significant differences in the confidence curve of the approximated prediction making it a good compromise for the method described herein.
- The PINN generates a continuous approximation in comparison to the grid as is evident in Figure 16. This can be a beneficial property when approximating parameters as the discretization creates a split distribution here which is not necessarily true to reality.
- The k-d tree evaluation is the most performant and given a computational grid of required resolution may perform as well as the simulator.
- The simulation component can be used to generate a high-resolution computation if required.
- When comparing the performance of the combined methods against simulator only, approximately the same posterior distributions may be generated in reduced time.

Further examples

The method for configuring and/or controlling a bioprocessing system performing a bioprocess is not limited to the above examples.

For example, each of simulator, PINN model, data storage structure, optimizer, probabilistic algorithm, etc. described above, may be realized by other suitable methods and/or structures.

Other embodiments may also include, but are not limited to, one or more of:

- employing another numerical solution method used in simulator, including any suitable discretization method for the partial differential equations or ordinary differential equation solver used for resulting discrete system of equations,
- employing another data storage structure, for example other data storage structure optimized for in memory-storage and search,
- employing another neural network structure of PINN model or employing other artificial intelligence models,
- employing another Bayesian sampler for the probabilistic algorithm. For example, any Bayesian sampler and in particular any Bayesian MCMC sampler can be used by the probabilistic algorithm;
- employing the method for configuration and/or control of bioprocessing systems other than a chromatography system or a bioreactor system.

Further, in the prediction phase any evaluation method can be used instead of simulation if the accuracy is sufficient for user requirements.

In addition, the physical model of a bioprocess performed by a bioprocessing system (bioprocessing model) is not limited to the above exemplary models and another known physical models may be used, for example employing a more refined or detailed physical model and/or models of other bioprocessing systems and/or bioprocesses.

For example, the physical model may also be a model describing a mass transfer in a bioreactor system comprising at least one bioreactor. One exemplary physical model describing the mass transfer in stirred microbioreactors is described in the article "Modeling mass transfer in stirred microbioreactors", Farsani et al. 2022. The model is a three-dimensional PDE mass transfer model for fluid motion in an agitated vessel, validated on an Ambr® 15 microbioreactor (Sartorius). The model may be used to predict fluid flow and transport inside two-phase bioreactors, for use in bioreactor scale-up, and to reduce the number of experiments needed in microscale bioreactors. The model can decompose gas transfer to the fluid into contributions from the free surface and individually sparged bubbles and identify the sensitivity of the mass transfer coefficient around each bubble to the local fluid mechanics. The important parameters in this model (model parameters) depend on the specific bioreactor being studied and the operating conditions used. In the case of the Ambr® 15 microbioreactor studied in this work, the important parameters include the gas flow rate, the agitation rate, the sparging method, the liquid volume, and the bubble size. Other important parameters may include the physical dimensions of the bioreactor, the properties of the fluids and gases involved, and any additional equipment or instrumentation used in the process. The model is designed to be self-consistent across operating conditions with no model reparameterization or tuning, which reduces the number of user-defined parameters and correlations needed. The model includes several parameters that need to be experimentally determined and have some variability, such as surface areas of bubbles. Variables like agitation, bubble size, velocities can have the effect of stressing cells with consequences to cell growth. The stress affects growth, which in turn affects output of the obtained biomass, leading to variations in titre, yield, profitability, productivity etc. Instead of obtaining point estimates for these final values, the method according to an aspect, its variations and examples of the invention can derive distributions of likely ranges which is a more realistic representation of what might be expected from this process.

Further, for example, the physical model may be composed of additional and/or other partial differential equations. In case of chromatography, the additional and/or other partial differential equations may, for example describe component concentrations in mobile phase along a column axis, mobile phase stagnant inside resin particles along radial axis and stationary phase along radial axis of a resin particle.

One way of finding parameters for this type of complex physical models may involve a staged estimation procedure that isolates specific parameters using a plurality of experiments. Below, an exemplary staged estimation procedure involving four types of experiments will be described in relation of a physical model of a chromatography system performing a chromatography process. Similar multistage procedures may also be used in connection with other types of bioprocessing system and bioprocesses, such as for example bioreactor systems and/or bioreactor processes.

1. In a first stage detached column experiments are performed to determine the band broadening effect of extra-column volumes. Neglecting extra column effects or even small errors in accounting for them can have a large impact on the binding parameters estimated at a later stage. In the model this would translate to adding some extra column-equations without binding but with unique axial dispersion coefficients.

2. The second stage involves isolating model parameters for characterizing the packed bed by setting the film diffusion coefficient to zero, which eliminates all but the column model equation from the system. This allows the approximation of the axial dispersion coefficient and column porosity. Experimentally, non-binding and non-pore penetrating tracer would be used for this purpose.

3. In a third stage, model parameters for characterizing the porous particles are isolated by setting the adsorption constants to zero, which eliminates certain equations from the system. This allows the approximation of kinetic rate constants.

4. In a fourth stage, the parameters of the binding model are estimated. This stage is typically the most complicated and time-consuming because of the inherent non-linearity of the more complex binding models required for describing preparative chromatography, especially for multi-component systems with competitive binding. To reduce correlations between the binding parameters, they are estimated from a set of several chromatograms that are measured at specifically designed operating conditions. Parameters vary widely with different choices of binding model.

In general, measurement data are generally prone to random and systematic errors that are in turn propagated to the estimated parameters. In the above stagewise procedure, parameters estimated in one stage are fixed in the next, and parameter errors are carried over to subsequent stages (see e.g., the article "Advanced score system and automated search strategies for parameter estimation in mechanistic chromatography modeling", (Lieres et al. 2021).

Against this background, the method for configuring and/or controlling a bioprocessing system (such as for example a chromatography system or a bioreactor system) performing a bioprocess (such as a chromatography process or a bioreactor process) solves at least one of the following problems of a conventional parameter determination:

- The problem concerning error propagation may be alleviated or eliminated by applying Bayesian parameter inference. Where the conventional way tries to find specific parameter values deterministically Bayesian parameter inference allows to use experimental data in a general way to find the distributions of parameters. These distributions take propagation errors from measurements into account. Furthermore, correlated parameters may be easily identified allowing design of experiments to break correlation structures.
- As discussed herein, finding parameters for bioprocessing modelling is a major undertaking. The staggered experiments require the same number of simulations to perform a deterministic parameter approximation. Extending this to a Bayesian parameter estimation increases the number of simulations into the thousands. Thus, a high-fidelity model run on the most expensive consumer hardware can take seconds, meaning that a complete procedure can extend to days or weeks even for a one-dimensional model. By combining various methods of solving the physical model (including one or more PDEs) and switching between the different methods as described herein, the computational time may be significantly reduced.

In the method for controlling and/or configuring a bioprocessing system (such as for example a chromatography system or a bioreactor system) and/or bioprocess, the physical model might be a general physical model describing a plurality of stages, such as a plurality of the above described stages (1) to (4). It is, however, possible that the physical model may be composed of a plurality of separate sub-models for each of the stages, each physical sub-model corresponding to a separate *physical model* for a subset of the stages (e.g., for one substage). Thus, each physical sub-model might constitute a subset of the complete set of equations making up the complete model. This may be regarded as equivalent to mathematically setting some parameters to zero in the system of equations. With this approach a setup phase (including PINN, grid, and simulator implementation), might be created for each stage. The parameter distributions might be collected for each separate stage. Finally, after completing a parameter estimation phase for each subset, a predictive phase might be started. In the predictive phase, a complete set of equations that would make up the physical model may be used and at this stage all parameter distributions would be generally known to accurately simulate this physical model. The benefits of such "deconstructed problem" approach might be computational (e.g., in terms of reduced computational time and/or resources), since it might be more efficient to calculate a subset problem than to calculate a "complete" problem. Furthermore, an analytic device or instrument might operate on a specific sub-stage and then the final predictive model might be used for example on a downstream processing device. The method for configuring and/or controlling a bioprocessing system and/or bioprocess described above may enable more correctly accessing the impact of assumptions that are made at each stage, thereby enabling improving of the performance of the bioprocessing system and/or bioprocess.

In addition, a physical model may be a one-dimensional model or may be a multidimensional model, which may more accurately describe advanced geometries of a bioprocessing system and/or advanced process workflows. Further, it might be necessary to make very large assumptions on geometry and/or workflow which do not reflect the many various consumables available for separation in downstream processing. The method for configuring and/or controlling a bioprocessing system and/or method described above may enable more correctly accessing the impact of assumptions regarding the geometries of a bioprocessing system and/or advanced process workflows, thereby enabling improving of the performance of the bioprocessing system and/or bioprocess.

The invention as described could be incorporated into a pc-based software product or as a computational component in an embedded hardware device. The PINN model could be trained in the device using local GPU or externally in cloud depending on implementation.

List of references

[1] Belbute-Peres, F. de A., Chen, Y. and Sha, F. (2021) 'HyperPINN: Learning parameterized differential equations with physics-informed hypernetworks'. arXiv. Available at: http://arxiv.org/abs/2111.01008 (Accessed: 22 August 2022).

[2] Briskot, T. et al. (2019) 'Prediction uncertainty assessment of chromatography models using Bayesian inference', Journal of Chromatography A, 1587, pp. 101-110. Available at: https://doi.org/10.1016/j.chroma.2018.11.076.

[3] Farsani, H.Y. et al. (2022) 'Modeling mass transfer in stirred microbioreactors', Chemical Engineering Science, 248, p. 117146. Available at: https://doi.org/10.1016/j.ces.2021.117146,

[4] Guiochon, G. et al. (2006) Fundamentals of preparative and nonlinear chromatography. Second edition. Amsterdam Boston Heidelberg London New York Oxford Paris San Diego San Francisco Singapore Sydney Tokyo: Elsevier Academic Press.

[5] Hastings, W.K. (1970) 'Monte Carlo sampling methods using Markov chains and their applications', Biometrika, 57(1), pp. 97-109. Available at: https://doi.org/10.1093/biomet/57.1.97.

[6] Heymann, W. et al. (2022) 'Advanced score system and automated search strategies for parameter estimation in mechanistic chromatography modeling', Journal of Chromatography A, 1661, p. 462693. Available at: https://doi.org/10.1016/j.chroma.2021.462693.

[7] Hoffman, M.D. and Gelman, A. (2011) 'The No-U-Turn Sampler: Adaptively Setting Path Lengths in Hamiltonian Monte Carlo'. Available at: https://doi.org/10.48550/ARXIV.1111.4246.

[8] Kingma, D.P. and Ba, J. (2014) 'Adam: A Method for Stochastic Optimization'. Available at: https://doi.org/10.48550/ARXIV.1412.6980.

[9] Kingma, D.P. and Welling, M. (2014) 'Auto-Encoding Variational Bayes'. arXiv. Available at: http://arxiv.org/abs/1312.6114 (Accessed: 24 August 2022).

[10] Liu, D.C. and Nocedal, J. (1989) 'On the limited memory BFGS method for large scale optimization', Mathematical Programming, 45(1-3), pp. 503-528. Available at: https://doi.org/10.1007/BF01589116.

[11] Seidel, S. et al. (2022) 'CFD modelling of a wave-mixed bioreactor with complex geometry and two degrees of freedom motion', Frontiers in Chemical Engineering, 4, p. 1021416. Available at: https://doi.org/10.3389/fceng.2022.1021416.

[12] Skilling, J. (2004) 'Nested Sampling', in AIP Conference Proceedings. BAYESIAN INFERENCE AND MAXIMUM ENTROPY METHODS IN SCIENCE AND ENGINEERING: 24th International Workshop on Bayesian Inference and Maximum Entropy Methods in Science and Engineering, Garching (Germany): AIP, pp. 395-405. Available at: https://doi.org/10.1063/1.1835238.

[13] Söderström, P. (2022) 'Physics-Informed Neural Networks for Liquid Chromatography'. Available at: https://www.diva-portal.org/smash/get/diva2:1672081/FULLTEXT01.pdf.

[14] Subraveti, S.G. et al. (2022) 'Can a computer "learn" nonlinear chromatography?: Physics-based deep neural networks for simulation and optimization of chromatographic processes', Journal of Chromatography A, 1672, p. 463037. Available at: https://doi.org/10.1016/j.chroma.2022.463037.

[15] Young, L.C. (2019) 'Orthogonal collocation revisited', Computer Methods in Applied Mechanics and Engineering, 345, pp. 1033-1076. Available at: https://doi.org/10.1016/j.cma.2018.10.019.

**List of Reference Numerals**

| | |
|---|---|
| 100 | method for configuring and/or controlling a chromatography system |
| S10 | setup phase |
| S11 | providing physical model |
| S12 | providing prior parameter distribution |
| S13 | providing an initial physics informed neural network model |
| S14 | providing an initial data storage |
| S15 | providing a simulation method |
| S16 | populating the initial data storage with simulated data |
| S17 | training the physics informed neural network model |
| 518 | initializing a probabilistic algorithm |
| S20 | parameter estimation phase |
| S211 | providing prior parameter distribution |
| S212 | providing observation data |
| S22 | starting probabilistic algorithm |
| S23 | selecting an evaluation method |
| S24 | continuing or switching the selected evaluation method |
| S25 | checking whether termination criteria for the probabilistic method are met |
| 526 | updating the data storage with new simulated results |
| S27 | retraining the physics informed neural network with new simulation results |
| S28 | determining a-posteriori parameter distribution(s) |
| S29 | propagating the at least one a-posteriori parameter distribution(s) to a prediction phase |
| S30 | prediction phase |
| S31 | obtaining at least one a-posteriori distribution |
| S32 | defining model conditions and |
| S33 | simulating output with credible intervals |
| 200 | a nested sampling algorithm (exemplary probabilistic algorithm) |
| S210 | providing a prior (i.e. the prior parameter distribution), |
| S220 | sampling the prior with a set of points |
| S230 | calculating the likelihood for the sampled points |
| S240 | conditional replacing of sample points with new sample points |
| S250 | forming a posterior sample by integration |
| S260 | termination of the nested sampling algorithm |

**Claims**

1. A computer-implemented method for configuring and/or controlling a bioprocessing system that is configured to physically perform and/or simulate a bioprocess, the method comprising:

   determining a-posteriori parameter distribution for model parameters of a physical model of the bioprocess by using a probabilistic algorithm, wherein said probabilistic algorithm uses observation data and a prior distribution of the model parameters, and wherein each model parameter corresponds to a state parameter of the bioprocessing system and/or bioprocess;
   generating, using samples of the determined a-posteriori parameter distribution, a predictive posterior distribution for a target set of model parameter values; and
   configuring and/or controlling at least one state parameter of the bioprocessing system and/or bioprocess based on the determined predictive posterior distribution, wherein the determining of the a-posteriori parameter distribution comprises:

   (i) selecting, based on predetermined criteria, an evaluation method from among at least the following evaluation methods:

   a simulation method configured to generate a solution to the physical model for a given set of values of the model parameters using a numerical method;

a search method configured to carry out a search for a nearest solution to the physical model for a given set of values of the model parameters in a data storage that stores a plurality precomputed solution-parameter sets, each precomputed solution-parameter set comprising a precomputed solution to the physical model generated by the simulation method and the respective set of model parameter values for which the precomputed solution was generated; and

an artificial intelligence based method configured to generate a solution to the physical model for a given set of values of the model parameters using a physics informed artificial intelligence model, which is trained based on the solutions generated by the simulation method and the physical model;

(ii) drawing samples from the prior distribution of the model parameters, each sample corresponding to a complete set of sought model parameters;

(iii) for each of the samples,

- carrying out an evaluation using the currently selected evaluation method to generate an approximate solution to the physical model for the current sample,
- computing the likelihood of the generated approximate solution based on the observed data and
- combining the computed likelihood with the sample to provide a draw of the a-posteriori parameter distribution;

(iv) if the computed likelihood does not fulfill predetermined error criteria, selecting a new evaluation method from among the simulation method, the search method and the artificial intelligence based method;

(v) iterating steps (ii) to (iv) until the a-posteriori parameter distribution reaches predetermined probabilistic algorithm criteria.

2. The computer-implemented method according to claim 1,

wherein in step (i), the search method, the artificial intelligence based method and the simulation method are selected in this order, and

wherein in step (iv) the new evaluation method is selected based on the following error criteria:

if the currently selected method is the artificial intelligence based method and if, after a predetermined number of iterations, the deviation between a solution provided by the artificial intelligence based method and a solution provided by simulation method exceeds a predetermined threshold, select the search method as the new artificial intelligence based method;

if the currently selected method is the search method and precision criteria required by the probabilistic algorithm fail, select a simulation method as the new artificial intelligence based method.

3. The computer-implemented method according to any one of the preceding claims, further comprising:

if the currently selected method is a simulation method, updating the data storage with at least one additional solution-parameter set comprising a solution to the physical model generated by the simulation method and the respective set of model parameters for which the solution was generated; and/or

if the currently selected method is a simulation method, retraining the physics informed artificial intelligence model based on at least one solution generated by the simulation method and the physical model.

4. The computer-implemented method according to any one of the preceding claims, further comprising:
parsing observation data into a numerical representation with common unit basis of the simulation method, the search method, and the artificial intelligence based method.

5. The computer-implemented method according to any one of the preceding claims, wherein the samples are drawn from the prior distribution of the model parameters using at least one of the following methods:

drawing samples from boundaries of a domain of interest;
randomly drawing samples;
uniformly drawing samples; and/or
drawing samples through Latin Hypercube Sampling.

6. The computer-implemented method of any one of the preceding claims, wherein the physical model includes a set of partial differential equations involving a plurality of variables, an unknown function dependent on the plurality of variables and partial derivatives of the function with respect to the variables, wherein each variable corresponds to a physical property of the bioprocessing system and/or bioprocess.

7. The computer-implemented method according to any one of the preceding claims, wherein

the probabilistic algorithm is a Bayesian inference algorithm;
the physics informed artificial intelligence model is a physics informed neural network model including at least one physics informed deep learning neural network; and/or
the numerical method includes a discretization method for solving partial or ordinary differential equations; and/or the bioprocessing system is a chromatography system that is configured to physically perform and/or simulate a chromatography process or a bioreactor system that is configured to physically perform and/or simulate a bioreactor process.

8. The computer-implemented method according to any one of the preceding claims, wherein the data storage is an in-memory tree-based data storage and/or wherein the search method is a tree-based search method.

9. The method according to any one of the preceding claims, further comprising a setup phase including:

generating by using the simulation method a plurality of solutions to the physical model for different values of model parameters and storing the generated solutions to the physical model and the respective set of model parameter values for which the solution was generated in the data storage; and
training an initially untrained physics informed artificial intelligence model based on the generated solutions to the physical model generated by the simulation method and the physical model.

10. The computer-implemented method according to any one of the preceding claims, wherein the configuring and/or controlling the bioprocessing system and/or bioprocess based on the determined predictive posterior distribution comprises:

generating prediction of a performance of the bioprocessing system and/or bioprocess for the target set of model parameter values based on the determined predictive posterior distribution; and/or
setting at least one state parameter of the bioprocessing system and/or bioprocess to the value of the corresponding model parameter included in the target set of model parameter values; and/or
controlling at least one state parameter of the bioprocessing system and/or bioprocess based on the value of the corresponding model parameter included in the target set of model parameter values.

11. The computer-implemented method according to claim 10, wherein the at least one state parameter of the bioprocessing system and/or bioprocess includes one or more of

parameters of at least one bioprocessing device configured to (physically) perform a certain part or stage of the bioprocess comprised in the bioprocessing system;
physical and/or chemical characteristics and/or quantity/amount of a consumable component of a bioprocessing system and/or bioprocess; and
physical and/or chemical characteristics at at least one specified point or portion in the bioprocessing system.

12. A method for performing a bioprocess on a bioprocessing system, comprising:
configuring and/or controlling the bioprocessing system according to the method of any one of the various aspects and embodiments described herein according to the method of any one of the preceding claims.

13. A computer program product comprising computer readable instructions that, when loaded and run on a computer, cause the computer to perform the method according to any one of claims 1 to 11.

14. A control device for configuring and/or controlling a bioprocessing system (10) that is configured to physically perform and/or simulate a bioprocess, the control device comprising at least one processor configured to perform the method according to any one of claims 1 to 11.

15. A system comprising:

a bioprocessing system that is configured to physically perform and/or simulate a bioprocess; and
the control device according to claim 14, the control device being connected to the bioprocessing system.

100

**SETUP PHASE** S10

S11 PHYSICAL MODEL

S15 CREATE SIMULATOR

S14 CREATE GRID STORAGE

S13 CREATE PINN

S12 PRIOR PARAMETER DISTRIBUTION

S16 POPULATE GRID

TRAIN PINN S17

S18 INITIALIZE PROBABILISTIC ALGORITHM

PARAMETER ESTIMATION PHASE

S20

**PARAMETER ESTIMATION PHASE** S20

S211 PRIOR PARAMETER DISTRIBUTION

OBSERVED DATA S212

PROBABILISTIC ALGORITHM S22

METHOD SELECTION S23

GRID QUERY | PINN | SIMULATOR

EVALUATE UNTIL SPECIFIC METHOD CRITERIA MET S24 S26

NO

S25 ALL CRITERIA SATISFIED

UPDATE GRID WITH NEW RESULTS

S27 RETRAIN PINN ON NEW INFORMATION

YES

S28 PARAMETER A-POSTERIORI DISTRIBUTION SAMPLING

S29 PREDICTION PHASE

**PREDICTION PHASE** S30

S31 A-POSTERIORI PARAMETER DISTRIBUTION

S32 CHANGE MODEL CONDITIONS

S33 SIMULATE OUTPUT WITH CREDIBILITY INTERVALS

**Fig. 1**

EP 4 492 164 A1

**Fig. 2A**

Fig. 2B

Fig. 2C

**Fig. 3**

$x$ vector of spatial coordinates

$t$ vector of temporal coordinates

$\hat{y}$ vector of approximate solution

$\zeta$ Operator parametrization

$\theta_\zeta$ vector network parameters

$\theta_\zeta^*$ vector of minimizing network parameters

EP 4 492 164 A1

NESTED SAMPLING ALGORITHM $\nearrow$ 200

```
┌─────────┐        ┌──────────────┐
│  PRIOR  │ ─────> │  LIKELIHOOD  │  S230
└─────────┘        └──────────────┘
  S210
            ┌──────────────────┐
            │  PRIOR SAMPLER   │  S220
            └──────────────────┘

            ┌──────────────────┐
            │     SAMPLER      │  S240
            └──────────────────┘

            ┌──────────────────┐
            │   INTEGRATION    │  S250
            └──────────────────┘

            ┌──────────────────┐
            │   TERMINATION    │  S260
            └──────────────────┘
```

**Fig. 4**

**Fig. 5**

**Fig. 6**

Fig. 7

**Fig. 8**

Fig. 9

DATA  MODEL  PREDICTION PHASE

PROBABILISTIC ALGORITHM

$\theta^* \longleftarrow$  $\longrightarrow \theta \longrightarrow$

$\tilde{y}$

$\hat{y}$

ERROR

$\tilde{y}$ vector of measured data

$\hat{y}$ vector of approximate solution

$\theta$ vector of parameters

$\theta^*$ vector of sampled parameters

Fig. 10

EP 4 492 164 A1

**Fig. 11**

**Fig. 12**

**Fig. 13**

**Fig. 14**

**Fig. 15**

**Fig. 16**

**Fig. 17**

**Fig. 18**

**Fig. 19**

**Fig. 20**

**Fig. 21**

**Fig. 22**

**Fig. 23**

**Fig. 24**

**Fig. 25**

**Fig. 26**

**Fig. 27**

**Fig. 28**

**Fig. 29**

**Fig. 30**

**Fig. 31**

**Fig. 32**

**Fig. 33**

Europäisches
Patentamt

European
Patent Office

Office européen
des brevets

# EUROPEAN SEARCH REPORT

**Application Number**

EP 23 29 0028

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 868 860 A1 (SARTORIUS STEDIM DATA ANALYTICS AB [SE]) 25 August 2021 (2021-08-25) * paragraphs [0057] – [0077]; figures 1-3 * * paragraph [0082]; figure 4 * * claims 1-15 * | 1-15 | INV. G05B13/02 C12M1/36 G01N35/00 G05B13/04 |
| X | TANJA HERNÁNDEZ RODRÍGUEZ ET AL: "Predicting industrial-scale cell culture seed trains-A Bayesian framework for model fitting and parameter estimation, dealing with uncertainty in measurements and model parameters, applied to a nonlinear kinetic cell culture model, using an MCMC method", BIOTECHNOLOGY AND BIOENGINEERING, JOHN WILEY, HOBOKEN, USA, vol. 116, no. 11, 2 September 2019 (2019-09-02), pages 2944-2959, XP071131683, ISSN: 0006-3592, DOI: 10.1002/BIT.27125 * the whole document * | 1-15 | |
| X,D | TILL BRISKOT ET AL: "Prediction uncertainty assessment of chromatography models using Bayesian inference", JOURNAL OF CHROMATOGRAPHY A, vol. 1587, 29 November 2018 (2018-11-29), pages 101-110, XP055755577, AMSTERDAM, NL ISSN: 0021-9673, DOI: 10.1016/j.chroma.2018.11.076 * the whole document * | 1-15 | TECHNICAL FIELDS SEARCHED (IPC) G05B G01N C12M |

The present search report has been drawn up for all claims

| Place of search | Date of completion of the search | Examiner |
|---|---|---|
| The Hague | 6 December 2023 | Itoafa, Alex |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 29 0028

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2023

| Patent document cited in search report | Publication date | Patent family member(s) | Publication date |
|---|---|---|---|
| EP 3868860 A1 | 25-08-2021 | CN 115175982 A | 11-10-2022 |
| | | EP 3868860 A1 | 25-08-2021 |
| | | US 2023068360 A1 | 02-03-2023 |
| | | WO 2021165480 A1 | 26-08-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **FARSANI et al.** *Modeling mass transfer in stirred microbioreactors*, 2022
- **LIERES et al.** *Advanced score system and automated search strategies for parameter estimation in mechanistic chromatography modeling*, 2021
- **BELBUTE-PERES, F. DE A.** ; **CHEN, Y.** ; **SHA, F.** HyperPINN: Learning parameterized differential equations with physics-informed hypernetworks. *arXiv.*, 2021, http://arxiv.org/abs/2111.01008
- **BRISKOT, T. et al.** Prediction uncertainty assessment of chromatography models using Bayesian inference. *Journal of Chromatography A*, 2019, vol. 1587, 101-110, https://doi.org/10.1016/j.chroma.2018.11.076
- **FARSANI, H.Y. et al.** Modeling mass transfer in stirred microbioreactors. *Chemical Engineering Science*, 2022, vol. 248, 117146, https://doi.org/10.1016/j.ces.2021.117146
- **GUIOCHON, G. et al.** Fundamentals of preparative and nonlinear chromatography. Elsevier Academic Press, 2006
- **HASTINGS, W.K.** Monte Carlo sampling methods using Markov chains and their applications. *Biometrika*, 1970, vol. 57 (1), 97-109, https://doi.org/10.1093/biomet/57.1.97
- **HEYMANN, W. et al.** Advanced score system and automated search strategies for parameter estimation in mechanistic chromatography modeling. *Journal of Chromatography A*, 2022, vol. 1661, 462693, https://doi.org/10.1016/j.chroma.2021.462693
- **HOFFMAN, M.D.** ; **GELMAN, A.** *The No-U-Turn Sampler: Adaptively Setting Path Lengths in Hamiltonian Monte Carlo*, 2011, https://doi.org/10.48550/ARXIV.1111.4246

- **KINGMA, D.P.** ; **BA, J.** *Adam: A Method for Stochastic Optimization*, 2014, https://doi.org/10.48550/ARXIV.1412.6980
- **KINGMA, D.P.** ; **WELLING, M.** Auto-Encoding Variational Bayes. *arXiv*, 2014, http://arxiv.org/abs/1312.6114
- **LIU, D.C.** ; **NOCEDAL, J.** On the limited memory BFGS method for large scale optimization. *Mathematical Programming*, 1989, vol. 45 (1-3), 503-528, https://doi.org/10.1007/BF01589116
- **SEIDEL, S. et al.** CFD modelling of a wave-mixed bioreactor with complex geometry and two degrees of freedom motion. *Frontiers in Chemical Engineering*, 2022, vol. 4, 1021416, https://doi.org/10.3389/fceng.2022.1021416
- Nested Sampling. **SKILLING, J.** AIP Conference Proceedings. BAYESIAN INFERENCE AND MAXIMUM ENTROPY METHODS IN SCIENCE AND ENGINEERING: 24th International Workshop on Bayesian Inference and Maximum Entropy Methods in Science and Engineering. AIP, 2004, 395-405
- **SÖDERSTRÖM, P.** *Physics-Informed Neural Networks for Liquid Chromatography*, 2022, https://www.diva-portal.org/smash/get/diva2:1672081/FULLTEXT01.pdf
- **SUBRAVETI, S.G. et al.** Can a computer "learn" nonlinear chromatography?: Physics-based deep neural networks for simulation and optimization of chromatographic processes. *Journal of Chromatography A*, 2022, vol. 1672, 463037, https://doi.org/10.1016/j.chroma.2022.463037
- **YOUNG, L.C.** Orthogonal collocation revisited. *Computer Methods in Applied Mechanics and Engineering*, 2019, vol. 345, 1033-1076, https://doi.org/10.1016/j.cma.2018.10.019